# EUROPEAN PATENT SPECIFICATION

(11) **EP 2 718 443 B1**
(45) Date of publication and mention of the grant of the patent: **29.11.2017**
(21) Application number: 12729391.8
(22) Date of filing: 30.05.2012
(51) Int. Cl.: C12N 15/82, C12N 5/04, C12N 5/10, C12N 9/16

(54) **METHODS AND MEANS TO MODIFY A PLANT GENOME AT A PRESELECTED SITE**
VERFAHREN UND VORRICHTUNG ZUR VERÄNDERUNG EINES PFLANZENGENOMS AN EINER VORAUSGEWÄHLTEN STELLE
MÉTHODES ET MOYENS POUR MODIFIER LE GÉNOME D'UNE PLANTE EN UN SITE PRÉSÉLECTIONNÉ

(30) Priority: 06.06.2011 US 201161493579 P; 06.06.2011 EP 11004570
(43) Date of publication of application: 16.04.2014
(73) Proprietor: Bayer CropScience NV, 1831 Diegem (BE)
(72) Inventor: D'HALLUIN, Katelijn, 9030 Mariakerke (BE)
(86) International application number: PCT/EP2012/060181
(87) International publication number: WO 2012/168124

(56) References cited:
- WO-A1-00/71733
- WO-A1-02/31167
- WO-A2-2004/006667
- WO-A2-2005/049842
- SHEN-JIE WU ET AL: "Enhanced Agrobacterium-mediated Transformation of Embryogenic Calli of Upland Cotton via Efficient Selection and Timely Subculture of Somatic Embryos", PLANT MOLECULAR BIOLOGY REPORTER, vol. 26, no. 3, 1 September 2008 (2008-09-01), pages 174-185, XP55035135, ISSN: 0735-9640, DOI: 10.1007/s11105-008-0032-9
- J. WU ET AL: "High-efficiency transformation of Gossypium hirsutum embryogenic calli mediated by Agrobacterium tumefaciens and regeneration of insect-resistant plants", PLANT BREEDING, vol. 124, no. 2, 1 April 2005 (2005-04-01) , pages 142-146, XP55035137, ISSN: 0179-9541, DOI: 10.1111/j.1439-0523.2004.01056.x
- TANVEER KHAN ET AL: "High-frequency regeneration via somatic embryogenesis of an elite recalcitrant cotton genotype (Gossypium hirsutum L.) and efficient Agrobacterium-mediated transformation", PLANT CELL, TISSUE AND ORGAN CULTURE, KLUWER ACADEMIC PUBLISHERS, DO, vol. 101, no. 3, 5 February 2010 (2010-02-05), pages 323-330, XP019831213, ISSN: 1573-5044
- RAJASEKARAN K ET AL: "High-frequency stable transformation of cotton (Gossypium hirsutum L.) by particle bombardment of embryogenic cell suspension cultures", PLANT CELL REPORTS, SPRINGER VERLAG, DE, vol. 19, no. 6, 1 May 2000 (2000-05-01), pages 539-545, XP002190392, ISSN: 0721-7714, DOI: 10.1007/S002990050770
- YANG Z ET AL: "Monitoring homologous recombination in rice (Oryza sativa L.)", MUTATION RESEARCH, ELSEVIER, AMSTERDAM, NL, vol. 691, no. 1-2, 10 September 2010 (2010-09-10), pages 55-63, XP027237811, ISSN: 0027-5107 [retrieved on 2010-07-27]

## Description

### FIELD OF THE INVENTION

The invention relates to the field of agronomy. More particularly, the invention provides methods and means to introduce a targeted modification, including insertion, deletion or substitution, at a precisely localized nucleotide sequence in the genome of a cotton plant using embryogenic callus.

### BACKGROUND ART

The need to introduce targeted modifications in plant genomes, including the control over the location of integration of foreign DNA in plants has become increasingly important, and several methods have been developed in an effort to meet this need (for a review see Kumar and Fladung, 2001, Trends in Plant Science, 6, pp155-159). These methods mostly rely on the initial introduction of a double stranded DNA break at the targeted location via expression of a double strand break inducing (DSBI) enzyme.

Activation of the target locus and/or repair or donor DNA through the induction of double stranded DNA breaks (DSB) via rare-cutting endonucleases, such as I-Scel has been shown to increase the frequency of homologous recombination by several orders of magnitude. (Puchta et al., 1996, Proc. Natl. Acad. Sci. U.S.A., 93, pp5055-5060; Chilton and Que, Plant Physiol., 2003; D'Halluin et al. 2008 Plant Biotechnol. J. 6, 93-102).

WO96/14408 describes an isolated DNA encoding the enzyme I-Scel. This DNA sequence can be incorporated in cloning and expression vectors, transformed cell lines and transgenic animals. The vectors are useful in gene mapping and site-directed insertion of genes.

WO00/46386 describes methods of modifying, repairing, attenuating and inactivating a gene or other chromosomal DNA in a cell through an I-Scel induced double strand break. Also disclosed are methods of treating or prophylaxis of a genetic disease in an individual in need thereof. Further disclosed are chimeric restriction endonucleases.

WO 2005/049842 describes methods and means to improve targeted DNA insertion in plants using rare-cleaving "double stranded break" inducing (DSBI) enzymes, as well as improved I-Scel encoding nucleotide sequences.

WO2006/105946 describes a method for the exact exchange in plant cells and plants of a target DNA sequence for a DNA sequence of interest through homologous recombination, whereby the selectable or screenable marker used during the homologous recombination phase for temporal selection of the gene replacement events can subsequently be removed without leaving a foot-print and without resorting to in vitro culture during the removal step, employing the therein described method for the removal of a selected DNA by microspore specific expression of a DSBI rare-cleaving endonuclease.

WO2008/037436 describe variants of the methods and means of WO2006/105946 wherein the removal step of a selected DNA fragment induced by a double stranded break inducing rare cleaving endonuclease is under control of a germline-specific promoter. Other embodiments of the method relied on non-homologous end-joining at one end of the repair DNA and homologous recombination at the other end. WO08/148559 describes variants of the methods of WO2008/037436, i.e. methods for the exact exchange in eukaryotic cells, such as plant cells, of a target DNA sequence for a DNA sequence of interest through homologous recombination, whereby the selectable or screenable marker used during the homologous recombination phase for temporal selection of the gene replacement events can subsequently be removed without leaving a foot-print employing a method for the removal of a selected DNA flanked by two nucleotide sequences in direct repeats.

WO 2003/004659 discloses recombination systems and to a method for removing nucleic acid sequences from the chromosomal DNA of eukaryotic organisms. The invention also relates to transgenic organisms (preferably plants), containing said systems or produced by said method.

WO 2006/032426 discloses improved recombination systems and methods for eliminating maker sequences from the genome of plants. Particularly, the invention is based on use of an expression cassette comprising the parsley ubiquitin promoter, and operably linked thereto a nucleic acid sequence coding for a sequence specific DNA-endonuclease.

WO 2009/006297 discloses methods and compositions for altering the genome of a monocot plant cell, and a monocot plant, involving the use a double-stranded break inducing agent to alter a monocot plant or plant cell genomic sequence comprising a recognition sequence for the double-stranded break inducing agent.

WO 2004/006667 describes improved methods of regeneration and Agrobacterium-mediated transformation of cotton via somatic embryogenesis.

WO 2005/103271 describes methods for high efficiency plant transformation via Agrobacterium-mediated T-DNA conjugation to suspension-cultured cells or calli, employing membranes of filters as porous solid support for the co-culture of T-DNA donor and recipient.

WO 2008/112633 relates to excision of explant material comprising meristematic tissue from cotton seeds. Methods for tissue preparation, storage, transformation, and selection or identification of transformed plants are disclosed, as are transformable meristem tissues and plants produced by such methods, and apparati for tissue preparation.

Methods genome engineering using a DSBI enzyme have been applied in plant like tobacco (see e.g. Puchta et al., 1996; Townsend et al. Nature 459:442-445, 2009) and corn (see e.g. WO 2009/006297, Shukla et al. Nature 459:437-441, 2009). However, there still remains a need for the development of methods for targeted genome modification of plants that are more recalcitrant in tissue culture and transformation, such as cotton. The present invention provides a contribution over the art by providing such methods, using cotton embryogenic callus for the introduction of an endonuclease alone or in combination with a repair DNA that is to be used as a template for double stranded DNA break repair.

### SUMMARY OF THE INVENTION

In a first embodiment, the invention provides a method for modifying the genome of a cotton plant cell at a predefined site comprising the steps of
a. inducing a double stranded DNA break in the vicinity or at of said predefined site, said double stranded break being induced by the introduction into said cell of a rare-cleaving endonuclease enzyme which recognizes a recognition sequence in the vicinity of or at said predefined site;
b. selecting a plant cell wherein said double stranded DNA break has been repaired resulting in a modification in the genome at said preselected site, wherein said modification is selected from
   i. a replacement of at least one nucleotide;
   ii. a deletion of at least one nucleotide;
   iii. an insertion of at least one nucleotide; or
   iv. any combination of i. - iii.;
   characterized in that said cell is comprised within friable embryogenic callus, wherein said friable embryogenic callus has been maintained under dim light conditions and on medium comprising active carbon.

In one embodiment, the endonuclease enzyme may be introduced into said cell by the delivery into said cell of a DNA molecule encoding said endonuclease enzyme.

In another embodiment, prior to step b. a foreign repair DNA molecule is delivered into said cell, said foreign repair DNA molecule being used as a template for repair of said double stranded DNA break.

In a specific embodiment, the embryogenic callus is induced from hypocotyl explants. Embryogenic callus may be induced on medium comprising active carbon. Prior to, during and after said introduction of said endonuclease enzyme, the embryogenic callus may be incubated in medium without hormones. The callus may also be incubated on solid medium prior to and after said introduction of said endonuclease enzyme. Further, during or after said introduction of the endonuclease enzyme, the embryogenic callus may be incubated for 1 to 4 days on a non-selective medium.

In one embodiment, the endonuclease enzyme encoding DNA and/or the foreign repair DNA is delivered into the cells of the embryogenic callus by particle bombardment. Bombardment may be performed with about 0.5 pmol foreign repair DNA and/or about 0.5 pmol endonuclease encoding DNA. Prior to bombardment the embryogenic callus may be incubated in a medium comprising 0.2M mannitol and 0.2M sorbitol for about 2 to about 20 hours. Further, during or after said introduction of said endonuclease enzyme, the embryogenic callus may be incubated for 1 to 4 days on a non-selective medium comprising 0.2 M mannitol.

In another embodiment, of the endonuclease enzyme encoding DNA and/or the foreign repair DNA is delivered into the cell of the embryogenic callus using Agrobacterium mediated transformation.Agrobacterium-mediated DNA transfer may be performed by co-culturing the callus with the Agrobacterium strain(s) comprising the DNA molecule(s) for about three days in a medium comprising 100 µM acetosyringone and/or 100 mg/l L-cysteine. After transformation, the calli may be incubated on a medium comprising 250mg/L or 125mg/L triacillin.

In a particular embodiment, the foreign repair DNA comprises at least one flanking nucleotide sequence having sufficient homology to the upstream or downstream DNA region of said predefined site to allow recombination with said upstream or downstream DNA region. The foreign repair DNA may comprise two flanking nucleotide sequences located on opposite ends of said foreign DNA, one of said flanking nucleotide sequence having sufficient homology to the upstream DNA region of said predefined site, the other flanking nucleotide sequence having sufficient homology to the downstream sequence of said predefined site to allow recombination between said flanking nucleotide sequences and said upstream and downstream DNA regions. The foreign repair DNA may also comprise a selectable marker gene and/or a plant expressible gene of interest. The plant expressible gene of interest can be selected from the group of a herbicide tolerance gene, an insect resistance gene, a disease resistance gene, an abiotic stress resistance gene, a gene encoding an enzyme involved in oil biosynthesis or carbohydrate biosynthesis, a gene encoding an enzyme involved in fiber strength or fiber length, a gene encoding an enzyme involved in biosynthesis of secondary metabolites.

In another embodiment, the foreign DNA consists of two flanking nucleotide sequences, one of said flanking nucleotide sequence having sufficient homology to the upstream DNA region of said predefined site, the other flanking nucleotide sequence having sufficient homology to the downstream sequence of said predefined site to allow recombination between said flanking nucleotide sequences and said upstream and downstream DNA regions.

In yet another embodiment, the preselected site is flanked by two regions with sufficient homology for recombination with each other.

The invention further provides methods for modifying the genome of a cotton plant cell at a predefined site using embryogenic callus as described above, wherein said cotton plant cell is further regenerated into a cotton plant. The thus generated cotton plant may be further crossed with another plant.

Also provided is a cotton plant cell comprising a modification at a predefined site of the genome, obtained by any of the method as described above, as well as a cotton plant or fiber or seed or propagating material of that plant, comprising a modification at a predefined site of the genome.

The invention further relates to a method of growing a cotton plant as described above, further comprising the step of applying a chemical to said plant or substrate wherein said plant is grown, as well as a method for producing a plant comprising a modification at a predefined site of the genome, comprising the step of crossing a plant consisting essentially of the plant cells as described above or a plant as described above with another plant or with itself and optionally harvesting seeds.

### FIGURE LEGENDS

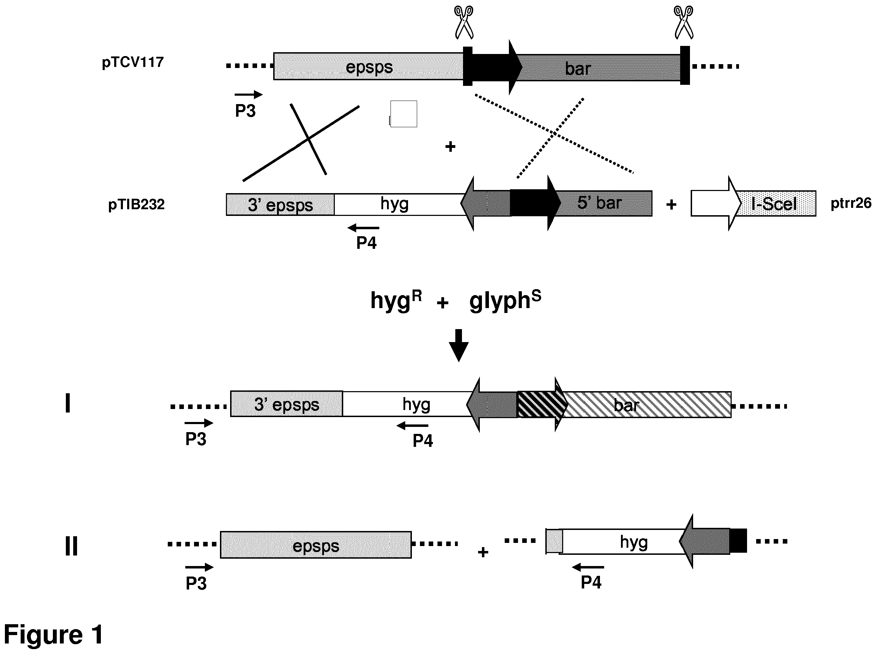

Figure 1: Schematic overview of targeted insertion/replacement through at least one-sided homologous recombination. Scissors indicate recognition sites for DSBI enzymes (I-Scel), block arrows represent promoters, arrows P3 and P4 represent primers, cross and dotted cross represent potential homologous recombination between the target construct pTCV117 and repair DNA pTIB323. After selection for hygromycin resistance (hygR) and glyphosate sensitivity (glyphS), two event groups can occur; (II) either (II) the repair DNA conferring hygromycin resistance is integrated into the genome at a random site and the EPSPS gene at the target locus is removed from bidirectional control of the 35S promoter through double stranded DNA break induction at the first (left) I-Scel site, in which case PCR amplification with primers P3 x P4 does not result in a PCR product, or (I) the EPSPS gene at the target locus is truncated through homologous recombination with the 3'EPSPS fragment of the repair DNA, whereby the hygromicin resistance gene is incorporated at the target locus, and whereby at the other end various scenarios are possible depending on which of the I-Scel sites is/are used (indicated by the striped patterning), in each case allowing the region between primers P3 and P4 to be amplified.

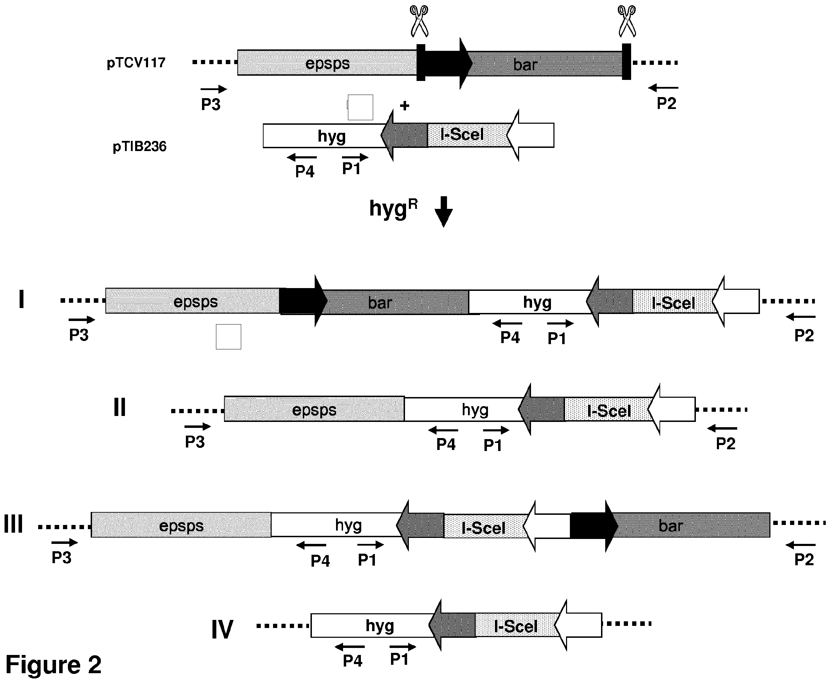

Figure 2: Schematic overview of targeted insertion/replacement through non-homologous end-joining. Selection for hygromycin resistance can result in either random integration of the repair DNA pTIB236 into the genome, whereby PCR amplification with primers P3 X P4 and P1 x P2 does not result in a PCR product, or (I-III) depending on which I-Scel site is used, insertion of the repair DNA at the site of DSBI (I and III) or replacement of the BAR gene by the repair DNA (II), resulting in PCR products with primers P3xP4 and P1xP2 of various lengths.

### DETAILED DESCRIPTION OF DIFFERENT EMBODIMENTS OF THE INVENTION

The inventors have found that targeted genome modification in cotton via double stranded DNA break induction can be efficiently performed by using friable embryogenic callus for the introduction of a double stranded break inducing (DSBI) enzyme, e.g. by introducing a DNA molecule encoding such and enzyme, and optionally a DNA molecule that functions as a template for repair of the double stranded DNA break, e.g. via direct DNA transfer methods (particle bombardment) or via Agrobacterium mediated DNA delivery. Using these delivery procedures into friable embryogenic callus, targeted insertions, replacements and deletions can be made in the nuclear genome of a cotton plant in the vicinity of the site of double stranded break induction.

As used herein, a rare-cleaving endonuclease is enzyme capable of inducing a DNA break at a particular nucleotide sequence, called the "recognition site" or "recognition sequence", i.e. they are site specific endonucleases. They are "rare-cleaving" in the sense that due to their specific, usually long (about 12-40 nt) recognition sites they have a very low frequency of cleaving, even in the larger plant genomes, e.g. they cut less than 20x, less than 10x, less than 5x or only one time in the target genome. Rare-cleaving double stranded DNA break inducing (DSBI) enzymes are rare-cleaving endonucleases that induce a double stranded DNA break (DSB) at their recognition site. Homing endonucleases, sometimes also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double stranded break in the intronless or inteinless alleles, these nucleases create recombinogenic ends, which engage in a gene conversion process that duplicates the coding sequence and leads to the insertion of an intron or an intervening sequence at the DNA level.

A well characterized homing endonuclease is I-Scel. I-Scel is a site-specific endonuclease, responsible for intron mobility in mitochondria in *Saccharomyces cerevisea.* The enzyme is encoded by the optional intron Sc LSU.1 of the 21S rRNA gene and initiates a double stranded DNA break at the intron insertion site generating a 4 bp staggered cut with 3'OH overhangs. The recognition site of I-Scel endonuclease extends over an 18 bp non-symmetrical sequence (Colleaux et al. 1988 Proc. Natl. Acad. Sci. USA 85: 6022-6026). The amino acid sequence for I-Scel and a universal code equivalent of the mitochondrial I-Scel gene have been provided by e.g. WO 96/14408. WO 96/14408 further discloses a number of variants of I-Scel protein which are still functional.

PCT application PCT/EP04/013122 (incorporated herein by reference) provides synthetic nucleotide sequence variants of I-Scel which have been optimized for expression in plants. The nucleotide sequence of such synthetic I-Sce I coding regions is set forth in SEQ ID No 1 in UIPAC code. The symbols of the UIPAC code have their usual meaning i.e. N=AorCorGorT; R=AorG;Y=CorT; B= C or G or T (not A); V= A or C or G (not T); D=A or G or T (not C); H=A or C or T (not G); K= G or T; M= A or C; S= G or C; W=A or T.

A list of other rare cleaving DSBI enzymes and their respective recognition sites is provided in Table I of WO 03/004659 (pages 17 to 20) (incorporated herein by reference). These include I-Sce I, I-Chu I, I-Dmo I, I-Cre I, I-Csm I, PI-Fli I, Pt-Mtu I, I-Ceu I, I-Sce II, I-Sce III, HO, PI-Civ I, PI-Ctr I, PI-Aae I, PI-BSU I, PI-Dhal, PI-Dra I, PI-Mav I, PI-Mch I, PI-Mfu I, PI-Mfl I, PI-Mga I, PI-Mgo I, PI-Min I, PI-Mka I, PI-Mle I, PI-Mma I, PI-Msh I, PI-Msm I, PI-Mth I, PI-Mtu I, PI-Mxe I, PI-Npu I, PI-Pfu I, PI-Rma I, PI-Spb I, PI-Ssp I, PI-Fac I, PI-Mja I, PI-Pho I, PI-Tag I, PI-Thy I, PI-Tko I or PI-Tsp I.

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the non-specific DNA-cleavage domain from a natural restriction enzyme, such as Fokl. Such enzymes are generally referred to Zinc finger endonucleases (ZFEs). Such methods have been described e.g. in WO 03/080809, WO94/18313 or WO95/09233 and in Isalan et al., 2001, Nature Biotechnology 19, 656- 660; Liu et al. 1997, Proc. Natl. Acad. Sci. USA 94, 5525-5530). Another way of producing custom-made rare-cleaving endonucleases or meganucleases, by selection from a library of variants, is described in WO2004/067736. Custom made meganucleases with altered sequence specificity and DNA-binding affinity may also be obtained through rational design as described in WO2007/047859. Another example of custom-designed rare-cleaving endonucleases include the so-called TALE nucleases, which are based on transcription activator-like effectors (TALEs) from the bacterial genus Xanthomonas fused to the catalytic domain of e.g. FOKI. The DNA binding specificity of these TALEs is defined by repeat-variable diresidues (RVDs) of tandem-arranged 34/35-amino acid repeat units, which can be modified to recognize specific target sequences (Christian et al., 2010, Genetics 186: 757-761, , WO11/072246, WO10/079430 and WO11/146121.. Such custom designed rare-cleaving endonucleases are also referred to as a non-naturally occurring endonucleases.

Since the re-designed meganucleases are derived from naturally occurring endonucleases, the available potential recognition sites are not entirely random but appear to have some degree of resemblance to the nucleotide sequence originally recognized by the naturally occurring endonuclease upon which the re-designed meganuclease is based. As stated by Gao et al (2010, The Plant Journal, pp 1-11) the structure-based protein design method to modify the DNA-binding characteristics of I-Crel are based on visual inspection of the I-Crel-DNA co-crystal structure leading to a prediction of a a large number of amino acid substitutions that change I-Crel base preference at particular positions in its recognition site. Individual amino acid substitutions were evaluated experimentally, and those that conferred the desired change in base preference were added to a database of mutations that can be "mixed and matched" to generate derivatives of I-Crel that recognize highly divergent DNA sites. In theory, the combinatorial diversity available using the current mutation database is sufficient to target an engineered endonuclease approximately every 1000 bp in a random DNA sequence.

Redesigned meganucleases can be based on the naturally occurring meganuclease I-Crel for use as a scaffold. I-Crel is a homing endonuclease found in the chloroplasts of *Chlamydomonas rheinhardti* (Thompson et al. 1992, Gene 119, 247-251). This endonuclease is a homodimer that recognizes a pseudo-palindromic 22 bp DNA site in the 23SrRNA gene and creates a double stranded DNA break that is used for the introduction of an intron. I-Crel is a member of a group of endonucleases carrying a single LAGLIDADG motif. LAGLIDADG enzymes contain one or two copies of the consensus motif. Single-motif enzymes, such as I-Crel function as homodimers, whereas double-motif enzymes are monomers with two separate domains. Accordingly, when re-designing meganucleases derived from an I-Crel scaffold to recognize a 22 bp nucleotide sequence of interest, two monomeric units are designed, each recognizing a part of the 22 bp recognition site, which are needed in concert to induce a double stranded break at the 22 bp recognition site(WO2007/047859). Concerted action may be achieved by linking the two monomeric units into one single chain meganuclease, or may also be achieved by promoting the formation of heterodimers, as described e.g. in WO2007/047859. Examples of such specifically designed meganucleases are described in e.g. EP10005926.0 and EP10005941.9 (unpublished).

Thus, for concerted action of such dimeric endonucleases, the subunits need to be dimerized in order to be able to induce a double stranded break at the preselected site in the genome. Enhanced concerted action may be achieved by linking the two monomeric units into one single chain meganuclease, or may also be achieved by promoting the formation of heterodimers, as described e.g. in WO2007/047859.

Various methods for DNA delivery into cells/tissues are known in the art, and include electroporation as illustrated in U.S. Pat. No. 5,384,253; microprojectile bombardment (biolistics) as illustrated in U.S. Patent Nos. 5,015,580; 5,550,318; 5,538,880; 6,160,208; 6,399,861 ; and 6,403,865; Agrobacterium-mediated transformation as illustrated in U.S. Patent Nos. 5,635,055; 5,824,877; 5,591,616; 5,981,840; and 6,384,301; protoplast transformation as illustrated in U.S. Patent No. 5,508,184, electroporation, chemically-assisted transformation, liposome- mediated transformation (see, e.g., A. Deshayes, et al. (1985) EMBO J. 4:2731-7.), carbon fiber, silicon carbide fiber or aluminum borate fiber (generally termed whiskers) (see, e.g., J. Brisibe, Exp. Bot. 51 (343):187-196 (2000); Dunwell (1999) Methods Mol. Biol. 1 11:375-82; and U.S. Patent No. 5,464,765), micro-injection (see, e.g., TJ. Reich, ef al. (1986) Biotechnology 4: 1001-1004) and viral-mediated transformation (see, e.g., S. B. Gelvin, (2005) Nat Biotechnol. 23:684-5), bombardment of plant cells with heterologous foreign DNA adhered to particles, ballistic particle acceleration, aerosol beam transformation (U.S. Patent Application No. 20010026941; U.S. Pat. No. 4,945,050; International Publication No. WO 91/00915; U.S. Patent Application No. 2002015066, WO 01/038514; Lec1 transformation, PEG transformation, and various other non-particle direct-mediated methods to transfer DNA.

Targeted genome modification of cotton cells for targeted genome modification according to the invention is performed on embryogenic callus, preferable friable callus. The term "callus" or "embryogenic callus" refers to a disorganized mass of mainly embryogenic cells and cell clusters produced as a consequence of plant tissue culture. Friable callus refers to callus with a friable texture with the potential to form shoots and roots and eventually regenerate into whole plants. Such callus can further be distinguished by a parrot-green/creamy color, readily dispersed cell clumps in liquid medium, and a nodular shape. Thus, as used herein "a plant cell comprised within embryogenic callus" refers to that cell being a callus cell itself, i.e. that cell being a part of the callus tissue.

Callus can be regenerated/induced from various tissue explants, such as hypocotyl, cotyledon, immature zygotic embryos, leaves, anthers, petals, ovules, roots, and meristems, stem cells and petioles. In one embodiment of the present invention, the explant is taken from the hypocotyl or cotyledon. In one embodiment, induction of embryogenic callus is performed by incubating the explants in medium comprising active carbon for about 2 to 4 months, preferably 4 months, or at least until embryogenic callus has been formed under dim light conditions.

In one embodiment, the calli are maintained on medium without hormones during the whole procedure of callus regeneration, DNA transfer and subsequent selection and regeneration. Hormones, as used herein refers to plant hormones such as auxins e.g. 2.4-D and cytokinins (e.g. Kin). In one embodiment, cells are maintained on solid medium during the whole procedure.

In another embodiment, after DNA delivery, the calli are maintained for 1-4 days, preferably 3 days, on a non-selective medium, i.e. a medium not containing a selection compound. The non-selective medium may comprise the components of the M100 substrate. After the 1-4 days on non-selective medium, the calli may be transferred to medium that may comprise the components of the M100 substrate and a selection compound. Using a selection compound after transformation allows for the enrichment of targeted recombination events in case a repair DNA is co-delivered which comprises a selectable marker gene conferring tolerance to the selection compound. After selection of embryogenic callus, embryo induction and embryo germination may take place on a selective medium that may comprise the components of the M104 substrate and active carbon. Further embryo development may take place on a non-selective substrate that may comprise the components of the M702 substrate and plant regeneration may take place on medium comprising the components of the M700 substrate. Components of the various substrates are described below.

As used herein, DNA delivery refers to the introduction of one ore more DNA molecules into a cell. This relates to both stable transfection, wherein the introduced DNA molecule is stably integrated into the genome of the host cell as well as the transient presence of those molecule(s) in the cell. It will be clear that for performing the methods of the invention, it is not required that the cells become stably transformed with the DNA encoding the endonuclease, but transient expression of the endonuclease may already be sufficient to induce the DNA double stranded break.

Various methods for DNA delivery into cells/tissues are known in the art, and include electroporation as illustrated in U.S. Pat. No. 5,384,253; microprojectile bombardment (biolistics) as illustrated in U.S. Patent Nos. 5,015,580; 5,550,318; 5,538,880; 6,160,208; 6,399,861 ; and 6,403,865; Agrobacterium-mediated transformation as illustrated in U.S. Patent Nos. 5,635,055; 5,824,877; 5,591,616; 5,981,840; and 6,384,301; protoplast transformation as illustrated in U.S. Patent No. 5,508,184, electroporation, chemically-assisted transformation, liposome- mediated transformation (see, e.g., A. Deshayes, et al. (1985) EMBO J. 4:2731-7.), carbon fiber, silicon carbide fiber or aluminum borate fiber (generally termed whiskers) (see, e.g., J. Brisibe, Exp. Bot. 51 (343):187-196 (2000); Dunwell (1999) Methods Mol. Biol. 1 11:375-82; and U.S. Patent No. 5,464,765), micro-injection (see, e.g., TJ. Reich, ef al. (1986) Biotechnology 4: 1001-1004) and viral-mediated transformation (see, e.g., S. B. Gelvin, (2005) Nat Biotechnol. 23:684-5), bombardment of plant cells with heterologous foreign DNA adhered to particles, ballistic particle acceleration, aerosol beam transformation (U.S. Patent Application No. 20010026941; U.S. Pat. No. 4,945,050; International Publication No. WO 91/00915; U.S. Patent Application No. 2002015066, WO 01/038514; Lec1 transformation, PEG transformation, and various other non-particle direct-mediated methods to transfer DNA.

In a specific embodiment, DNA delivery into callus comprising the cotton cells according to the invention is performed by direct DNA transfer methods, such as particle bombardment.

In one embodiment, prior to particle bombardment, calli are preplasmolysed in medium comprising mannitol and sorbitol for about 2 to about 20 hours, preferably about 2 to 4 hours.

In another specific embodiment delivery of DNA into cotton cells according to the invention is performed by Agrobacterium mediated transformation. In one embodiment, embryogenic calli are contacted with an Agrobacterium strain containing the DNA to be introduced in the cotton cells, after which the calli are co-cultivated with the Agrobacterium strain in medium comprising acetosyringone and L-cysteine for about 3 days in the dark.

In another embodiment, after the co-cultivation, transformed embryogenic calli are selected on a selection medium (i.e. comprising one or more selection compounds) further comprising triacillin.

It will be understood that the endonuclease encoding DNA and foreign repair DNA can be co-delivered to the cell or tissue (e.g. callus) sequentially (or reverse sequentially), using the same or different delivery methods, or they can be co-delivered simultaneously, e.g. whereby the foreign repair DNA and the endonuclease encoding DNA are comprised within the same mixture or even in the same molecule.

The endonuclease enzyme may but need not comprise a nuclear localization signal (NLS) (Raikhel, Plant Physiol. 100: 1627-1632 (1992) and references therein), such as the NLS of SV40 large T-antigen (Kalderon et al. Cell 39: 499-509, 1984). The nuclear localization signal may be located anywhere in the protein, but is conveniently located at the N-terminal end of the protein. The nuclear localization signal may replace one or more of the amino acids of the double stranded break inducing enzyme.

The induction of a double stranded break at a preselected site allows several potential applications. If no foreign repair DNA is introduced, the DNA region near the endonuclease recognition site may be altered by deletion, replacement or insertion of one or several to many nucleotides. In that way, the formation of small or larger deletions or insertions at the preselected site can for example inactivate the gene comprising the nucleotide sequence of the preselected site/recognition site. If the genomic DNA regions located upstream and downstream of the preselected site or recognition site have sufficient homology to each other to allow recombination between the upstream and downstream DNA region, the intervening DNA region, i.e. the DNA region between the two homologous upstream and downstream DNA region may be deleted (looped out). This can for example be used to remove previously introduced sequences such as marker genes, as e.g. described in WO 06/105946.

If the double stranded DNA break induction is accompanied by the introduction of a foreign repair DNA molecule which is used as a template, the double stranded break repair can occur basically in three ways. The repair DNA can be integrated into the genomic DNA at the DSB site by non-homologous end joining at both ends, or if one or two flanking regions with homology to the up- and/or downstream regions of the preselected site are present in the repair DNA, integration of the repair DNA can also occur (partly) through homologous recombination. As such, the double stranded break at the preselected site will also facilitate replacement of a DNA region in the vicinity of that site for a DNA region of interest e.g. as described in WO 06/105946, WO08/037436 or WO08/148559.

To insert a foreign DNA by homologous recombination at the preselected site, the foreign DNA may comprise at least one flanking DNA region having a nucleotide sequence which is similar to the nucleotide sequence of the DNA region upstream or downstream of the preselected site. The foreign DNA may also comprise two flanking DNA regions, located on opposite ends of the molecule and which have sufficient homology to nucleotide sequence of the DNA region upstream and downstream of the preselected site respectively to allow recombination between said flanking regions and said upstream and downstream region.

As used herein "a preselected site" or "predefined site" indicates a particular nucleotide sequence in the plant nuclear genome, located in or near the target DNA sequence at which location it is desired to insert the foreign DNA or to exchange the target DNA sequence. A person skilled in the art would be able to either choose a double stranded DNA break inducing ("DSBI") enzyme recognizing the selected target nucleotide sequence or engineer such a DSBI endonuclease. Alternatively, a DSBI endonuclease recognition site may be introduced into the plant genome using any conventional transformation method or by conventional breeding using a plant line having a DSBI endonuclease recognition site in its genome, and any desired foreign DNA may afterwards be introduced into that previously introduced preselected target site.

As used herein "located in the vicinity" refers to the site of double DNA stranded break induction, i.e. the recognition site of the endonuclease, being located at a distance of between 500 bp, 1 kbp, 2kbp, 3kbp, 4kbp, 5kbp to 10kbp from the predefined site, i.e. the site in the genomic DNA which is to be modified (the target site).

As used herein "a flanking DNA region" is a DNA with a nucleotide sequence having homology to the DNA regions respectively upstream and/or downstream of the target DNA sequence or preselected site. This allows to better control the precision of the intended modification. Indeed, integration by homologous recombination will allow precise joining of the foreign DNA fragment to the plant nuclear genome up to the nucleotide level.

To have sufficient homology for recombination, the flanking DNA regions of the repair DNA may vary in length, and should be at least about 10 nucleotides in length. However, the flanking region may be as long as is practically possible (e.g. up to about 100-150 kb such as complete bacterial artificial chromosomes (BACs). Preferably, the flanking region will be about 50 bp to about 2000 bp. Moreover, the regions flanking the foreign DNA of interest need not be identical to the DNA regions flanking the preselected site and may have between about 80% to about 100% sequence identity, preferably about 95% to about 100% sequence identity with the DNA regions flanking the preselected site. The longer the flanking region, the less stringent the requirement for homology. Furthermore, it is preferred that the sequence identity is as high as practically possible in the vicinity of the DSB. Furthermore, to achieve exchange of the target DNA sequence without changing the DNA sequence of the adjacent DNA sequences, the flanking DNA sequences should preferably be identical to the upstream and downstream DNA regions flanking the preselected site or the target DNA sequence destined to be exchanged. The same criteria apply for recombination between the upstream and downstream region bearing homology to each other to remove the intervening DNA sequences.

Moreover, the regions flanking the foreign DNA of interest need not have homology to the regions immediately flanking the preselected site, but may have homology to a DNA region of the genome further remote from that preselected site. Homologous recombination between the genomic DNA and the repair DNA will then result in a removal of the target DNA between the preselected insertion site and the DNA region of homology. In other words, the target DNA located between the homology regions will be substituted for the foreign DNA between the flanking regions. When the repair DNA consists of the two flanking sequences only, i.e. lacking any intervening sequences, this approach can be used to specifically delete the genomic region located between the two homology regions.

It will be clear that, in the case where homology regions are present in the foreign repair DNA, also site-specific recombinases can be used to carry out the methods of the invention. Site-specific recombinases require two recognition sites, which can be located on the same DNA molecule but also on two different DNA molecules, between which recombination occurs. Thus, a repair DNA comprising at least one such recognition site can be targeted to a genomic locus also comprising at least one such site. Examples of site-specific recombinases are well known in the art and include for instance the Cre-Lox system from bacteriophage P1 (Austin et al., 1981, Cell, 25:729-736), the Flp-Frt system from Saccheromyces, cerevisiae (Broach et al., 1982, Cell, 29:227-234), the R-RS system from Zygosaccharomyces rouxii (Araki et a1., 1985,J. MoL Biol.,182: 191-203) and the integrase from the Streptomyces phage PhiC31 (Thorpe & Smith, 1998, Proc. Natl. Acad. Sci., 95: 5505-5510; Groth et al., 2000, Proc. Natl. Acad. Sci., 97: 5995-6000).

The foreign DNA may also comprise a selectable or screenable marker, which may or may not be removed after insertion, as e.g. described in WO06/105946, WO08/037436 and WO08/148559.

Selectable or screenable markers" as used herein have there usual meaning in the art and include, but are not limited to plant expressible phosphinotricin acetyltransferase, neomycine phosphotransferase, glyphosate oxidase, glyphosate tolerant EPSP enzyme, nitrilase gene, mutant acetolactate synthase or acetohydroxyacid synthase gene, β-glucoronidase (GUS), R-locus genes, green fluorescent protein and the likes. Selectable markers may provide tolerance or resistance to selection compounds such as phosphinotricin, neomycin, glyphosate, hygromicin, ALS-inhibiting herbicides (e.g. sulphonyl urea and the like) or may otherwise provide means for selecting or enriching for cells wherein the desired modification has taken place, e.g. by visual means (GUS staining, fluorescence).

The selection of the plant cell or plant wherein the selectable or screenable marker and the rest of the foreign DNA molecule has been introduced by homologous recombination through the flanking DNA regions can e.g. be achieved by screening for the absence of sequences present in the transforming DNA but located outside of the flanking DNA regions. Indeed, presence of sequences from the transforming DNA outside the flanking DNA regions would indicate that the origination of the transformed plant cells is by random DNA insertion. To this end, selectable or screenable markers may be included in the transforming DNA molecule outside of the flanking DNA regions, which can then be used to identify those plant cells which do not have the selectable or screenable markers located outside of the transforming DNA and which may have arisen by homologous recombination through the flanking DNA regions. Alternatively, the transforming DNA molecule may contain selectable markers outside the flanking DNA regions that allow selection for the absence of such genes (negative selectable marker genes).

It will be clear that the methods according to the invention allow insertion of any DNA of interest including DNA comprising a nucleotide sequence with a particular nucleotide sequence signature e.g. for subsequent identification. The DNA of interest may also be one or more plant expressible gene(s) including but not limited to a herbicide tolerance gene, an insect resistance gene, a disease resistance gene, an abiotic stress resistance gene,
a gene encoding an enzyme involved in oil biosynthesis or carbohydrate biosynthesis, a gene encoding an enzyme involved in fiber strength or fiber length, a gene encoding an enzyme involved in biosynthesis of secondary metabolites.

Herbicide-tolerance genes include a gene encoding the enzyme 5-enolpyruvylshikimate-3-phosphate synthase (EPSPS). Examples of such EPSPS genes are the AroA gene (mutant CT7) of the bacterium Salmonella typhimurium (Comai et al., 1983, Science 221, 370-371), the CP4 gene of the bacterium Agrobacterium sp. (Barry et al., 1992, Curr. Topics Plant Physiol. 7, 139-145), the genes encoding a Petunia EPSPS (Shah et al., 1986, Science 233, 478-481), a Tomato EPSPS (Gasser et al., 1988, J. Biol. Chem. 263, 4280-4289), or an Eleusine EPSPS (WO 01/66704). It can also be a mutated EPSPS as described in for example EP 0837944, WO 00/66746, WO 00/66747 or WO02/26995. Glyphosate-tolerant plants can also be obtained by expressing a gene that encodes a glyphosate oxido-reductase enzyme as described in U.S. Patent Nos. 5,776,760 and 5,463,175. Glyphosate-tolerant plants can also be obtained by expressing a gene that encodes a glyphosate acetyl transferase enzyme as described in for example WO 02/36782, WO 03/092360, WO 05/012515 and WO 07/024782. Glyphosate-tolerant plants can also be obtained by selecting plants containing naturally-occurring mutations of the above-mentioned genes, as described in for example WO 01/024615 or WO 03/013226. EPSPS genes that confer glyphosate tolerance are described in e.g. US Patent Application Nos 11/517,991, 10/739,610, 12/139,408, 12/352,532, 11/312,866, 11/315,678, 12/421,292, 11/400,598, 11/651,752, 11/681,285, 11I605,824, 12/468,205, 111760,570, 111762,526, 111769,327, 11/769,255, 11/943801 or 12/362,774. Other genes that confer glyphosate tolerance, such as decarboxylase genes, are described in e.g. US patent applications 11/588,811, 111185,342, 12/364,724, 11/185,560 or 12/423,926.

Other herbicide tolereance genes may encode an enzyme detoxifying the herbicide or a mutant glutamine synthase enzyme that is resistant to inhibition, e.g. described in US Patent Application No 11/760,602. One such efficient detoxifying enzyme is an enzyme encoding a phosphinothricin acetyltransferase (such as the bar or pat protein from Streptomyces species). Phosphinothricin acetyltransferases are for example described in U.S. Patent Nos. 5,561,236; 5,648,477; 5,646,024; 5,273,894; 5,637,489; 5,276,268; 5,739,082; 5,908,810 and 7,112,665.

Herbicide-tolerance genes may also confer tolerance to the herbicides inhibiting the enzyme hydroxyphenylpyruvatedioxygenase (HPPD). Hydroxyphenylpyruvatedioxygenases are enzymes that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated or chimeric HPPD enzyme as described in WO 96/38567, WO 99/24585, and WO 99/24586, WO 2009/144079, WO 2002/046387, or US 6,768,044. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme having prephenate deshydrogenase (PDH) activity in addition to a gene encoding an HPPD-tolerant enzyme, as described in WO 2004/024928. Further, plants can be made more tolerant to HPPD-inhibitor herbicides by adding into their genome a gene encoding an enzyme capable of metabolizing or degrading HPPD inhibitors, such as the CYP450 enzymes shown in WO 2007/103567 and WO 2008/150473.

Still further herbicide tolerance genes encode variant ALS enzymes (also known as acetohydroxyacid synthase, AHAS) as described for example in Tranel and Wright (2002, Weed Science 50:700-712), but also, in U.S. Patent No. 5,605,011, 5,378,824, 5,141,870, and 5,013,659. The production of sulfonylurea-tolerant plants and imidazolinone-tolerant plants is described in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270. Other imidazolinone-tolerance genes are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerance gebnes are described in for example WO 07/024782 and US Patent Application No 61/288958.

Insect resistance gene may comprising a coding sequence encoding:
1) an insecticidal crystal protein from Bacillus thuringiensis or an insecticidal portion thereof, such as the insecticidal crystal proteins listed by Crickmore et al. (1998, Microbiology and Molecular Biology Reviews, 62: 807-813), updated by Crickmore et al. (2005) at the Bacillus thuringiensis toxin nomenclature, online at: http://www.lifesci.sussex.ac.uk/Home/Neil_Crickmore/Bt/), or insecticidal portions thereof, e.g., proteins of the Cry protein classes Cry1Ab, Cry1Ac, Cry1 B, Cry1 C, Cry1D, Cry1 F, Cry2Ab, Cry3Aa, or Cry3Bb or insecticidal portions thereof (e.g. EP 1999141 and WO 2007/107302), or such proteins encoded by synthetic genes as e.g. described in and US Patent Application No 12/249,016 ; or
2) a crystal protein from Bacillus thuringiensis or a portion thereof which is insecticidal in the presence of a second other crystal protein from Bacillus thuringiensis or a portion thereof, such as the binary toxin made up of the Cry34 and Cry35 crystal proteins (Moellenbeck et al. 2001, Nat. Biotechnol. 19: 668-72; Schnepf et al. 2006, Applied Environm. Microbiol. 71, 1765-1774) or the binary toxin made up of the Cry1A or Cry1 F proteins and the Cry2Aa or Cry2Ab or Cry2Ae proteins (US Patent Appl. No. 12/214,022 and EP 08010791.5); or
3) a hybrid insecticidal protein comprising parts of different insecticidal crystal proteins from Bacillus thuringiensis, such as a hybrid of the proteins of 1) above or a hybrid of the proteins of 2) above, e.g., the Cry1A.105 protein produced by corn event MON89034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or
5) an insecticidal secreted protein from Bacillus thuringiensis or Bacillus cereus, or an insecticidal portion thereof, such as the vegetative insecticidal (VIP) proteins listed at: http://www.lifesci.sussex.ac.uk/home/Neil_Crickmore/Bt/vip.html, e.g., proteins from the VIP3Aa protein class; or
6) a secreted protein from Bacillus thuringiensis or Bacillus cereus which is insecticidal in the presence of a second secreted protein from Bacillus thuringiensis or B. cereus, such as the binary toxin made up of the VIP1A and VIP2A proteins (WO 94/21795); or
7) a hybrid insecticidal protein comprising parts from different secreted proteins from Bacillus thuringiensis or Bacillus cereus, such as a hybrid of the proteins in 1) above or a hybrid of the proteins in 2) above; or
8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102; or
9) a secreted protein from Bacillus thuringiensis or Bacillus cereus which is insecticidal in the presence of a crystal protein from Bacillus thuringiensis, such as the binary toxin made up of VIP3 and Cry1A or Cry1F (US Patent Appl. No. 61/126083 and 61/195019), or the binary toxin made up of the VIP3 protein and the Cry2Aa or Cry2Ab or Cry2Ae proteins (US Patent Appl. No. 12/214,022 and EP 08010791.5);
10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein).

An "insect-resistant gene as used herein, further includes transgenes comprising a sequence producing upon expression a double-stranded RNA which upon ingestion by a plant insect pest inhibits the growth of this insect pest, as described e.g. in WO 2007/080126, WO 2006/129204, WO 2007/074405, WO 2007/080127 and WO 2007/035650.

Abiotic stress tolerance genes include
1) a transgene capable of reducing the expression and/or the activity of poly(ADP-ribose) polymerase (PARP) gene in the plant cells or plants as described in WO 00/04173, WO/2006/045633, EP 04077984.5, or EP 06009836.5.
2) a transgene capable of reducing the expression and/or the activity of the PARG encoding genes of the plants or plants cells, as described e.g. in WO 2004/090140.
3) a transgene coding for a plant-functional enzyme of the nicotineamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP 04077624.7, WO 2006/133827, PCT/EP07/002433, EP 1999263, or WO 2007/107326.

Enzymes involved in carbohydrate biosynthesis include those described in e.g. EP 0571427, WO 95/04826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, EP 06090134.5, EP 06090228.5, EP 06090227.7, EP 07090007.1, EP 07090009.7, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026 or WO 97/20936 or enzymes involved in the production of polyfructose, especially of the inulin and levan-type, as disclosed in EP 0663956, WO 96/01904, WO 96/21023, WO 98/39460, and WO 99/24593, the production of alpha-1,4-glucans as disclosed in WO 95/31553, US 2002031826, US 6,284,479, US 5,712,107, WO 97/47806, WO 97/47807, WO 97/47808 and WO 00/14249, the production of alpha-1,6 branched alpha-1,4-glucans, as disclosed in WO 00/73422, the production of alternan, as disclosed in e.g. WO 00/47727, WO 00/73422, EP 06077301.7, US 5,908,975 and EP 0728213, the production of hyaluronan, as for example disclosed in WO 2006/032538, WO 2007/039314, WO 2007/039315, WO 2007/039316, JP 2006304779, and WO 2005/012529.

The person skilled in the art will appreciate that, in addition to the nuclear genome, the methods of the invention may also be applied to modify e.g. the chloroplast genome or mitochondrial genome, whereby DSB induction at the predefined site and can further be enhanced by providing the correct targeting signal to the endonuclease enzyme.

Also disclosed are cotton plant cells and plants generated according to the methods of the invention. Such plants may contain a heterologous or foreign DNA sequence inserted at or instead of a target sequence or may contain a deletion, and will only be different from their progenitor plants by the presence of the particular modification.

As disclosed, the plant cells of the invention, i.e. a plant cell comprising the T-DNA combination as well as plant cells generated according to the methods disclosed herein comprising the intended genomic modification, may be non-propagating cells.

The cotton plants obtained by the methods described herein may be further crossed by traditional breeding techniques with other plants to obtain progeny plants comprising the targeted modification.

The cotton plants and seeds disclosed herein may be further treated with a chemical compound, such as a chemical compound selected from the following lists:
- Herbicides: Diuron, Fluometuron, MSMA, Oxyfluorfen, Prometryn, Trifluralin, Carfentrazone, Clethodim, Fluazifop-butyl, Glyphosate, Norflurazon, Pendimethalin, Pyrithiobac-sodium, Trifloxysulfuron, Tepraloxydim, Glufosinate, Flumioxazin, Thidiazuron
- Insecticides: Acephate, Aldicarb, Chlorpyrifos, Cypermethrin, Deltamethrin, Abamectin, Acetamiprid, Emamectin Benzoate, Imidacloprid, Indoxacarb, Lambda-Cyhalothrin, Spinosad, Thiodicarb, Gamma-Cyhalothrin, Spiromesifen, Pyridalyl, Flonicamid Flubendiamide, Triflumuron, Rynaxypyr, Beta-Cyfluthrin, Spirotetramat, Clothianidin, Thiamethoxam, Thiacloprid, Dinetofuran, Flubendiamide, Cyazypyr, Spinosad, Spinotoram, gamma Cyhalothrin, 4-[[(6-Chlorpyridin-3-yl)methyl](2,2-difluorethyl)amino]furan-2(5H)-on, Thiodicarb, Avermectin, Flonicamid, Pyridalyl, Spiromesifen, Sulfoxaflor
- Fungicides: Azoxystrobin, Bixafen, Boscalid, Carbendazim, Chlorothalonil, Copper, Cyproconazole, Difenoconazole, Dimoxystrobin, Epoxiconazole, Fenamidone, Fluazinam, Fluopyram, Fluoxastrobin, Fluxapyroxad, Iprodione, Isopyrazam, Isotianil, Mancozeb, Maneb, Metominostrobin, Penthiopyrad, Picoxystrobin, Propineb, Prothioconazole, Pyraclostrobin, Quintozene, Tebuconazole, Tetraconazole, Thiophanate-methyl, Trifloxystrobin

Cotton, as used herein refers to any existing cotton variety. For example, the cotton plant cell can be from a variety useful for growing cotton. The most commonly used cotton varieties are Gossypium barbadense, G. hirsutum, G. arboreum and G. herbaceum. Further varieties include G. africanum and G. raimondii.

Examples of cotton plants disclosed herein include those from which embryogenic callus can be derived, such as Coker 312, Coker310, Coker 5Acala SJ-5, GSC25110, FIBERMAX 819 , Siokra 1-3, T25, GSA75, Acala SJ2, Acala SJ4, Acala SJ5, Acala SJ-C1, Acala B1644, Acala B1654-26, Acala B1654-43, Acala B3991, Acala GC356, Acala GC510, Acala GAM1, Acala C1, Acala Royale, Acala Maxxa, Acala Prema, Acala B638, Acala B1810, Acala B2724, Acala B4894, Acala B5002, non Acala "picker" Siokra, "stripper" variety FC2017, Coker 315, STONEVILLE 506, STONEVILLE 825, DP50, DP61, DP90, DP77, DES119, McN235, HBX87, HBX191, HBX107, FC 3027, CHEMBRED A1, CHEMBRED A2, CHEMBRED A3, CHEMBRED A4, CHEMBRED B1, CHEMBRED B2, CHEMBRED B3, CHEMBRED C1, CHEMBRED C2, CHEMBRED C3, CHEMBRED C4, PAYMASTER 145, HS26, HS46, SICALA, PIMA S6 ORO BLANCO PIMA, FIBERMAX FM5013, FIBERMAX FM5015, FIBERMAX FM5017, FIBERMAX FM989, FIBERMAX FM832, FIBERMAX FM966, FIBERMAX FM958, FIBERMAX FM989, FIBERMAX FM958, FIBERMAX FM832, FIBERMAX FM991, FIBERMAX FM819, FIBERMAX FM800, FIBERMAX FM960, FIBERMAX FM966, FIBERMAX FM981, FIBERMAX FM5035, FIBERMAX FM5044, FIBERMAX FM5045, FIBERMAX FM5013, FIBERMAX FM5015, FIBERMAX FM5017 or FIBERMAX FM5024 and plants with genotypes derived thereof. These are suitable for applying the methods a described above.

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined may comprise additional DNA regions etc.

The term "plant" also includes progeny of plants which retain the distinguishing characteristics of the parents, such as seed obtained by selfing or crossing, e.g. hybrid seed, hybrid plants and plant parts derived therefrom.

As used herein, "plant part" includes any plant organ or plant tissue, including but not limited to fruits, seeds, embryos, fibers, meristematic regions, callus tissue, leaves, roots, shoots, flowers, gametophytes, sporophytes, pollen, and microspores.

The terms "protein" or "polypeptide" as used herein describes a group of molecules consisting of more than 30 amino acids, whereas the term "peptide" describes molecules consisting of up to 30 amino acids. Proteins and peptides may further form dimers, trimers and higher oligomers, i.e. consisting of more than one (poly)peptide molecule. Protein or peptide molecules forming such dimers, trimers etc. may be identical or non-identical. The corresponding higher order structures are, consequently, termed homo- or heterodimers, homo- or heterotrimers etc. The terms "protein" and "peptide" also refer to naturally modified proteins or peptides wherein the modification is effected e.g. by glycosylation, acetylation, phosphorylation and the like. Such modifications are well known in the art.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other, is regarded as a position with non-identical residues. The alignment of the two sequences is performed by the Needleman and Wunsch algorithm (Needleman and Wunsch 1970). The computer-assisted sequence alignment above, can be conveniently performed using standard software program such as GAP which is part of the Wisconsin Package Version 10.1 (Genetics Computer Group, Madison, Wisconsin, USA) using the default scoring matrix with a gap creation penalty of 50 and a gap extension penalty of 3.

The sequence listing contained in the file named ,,"BCS11-2008_WO_ST25", which is 59 kilobytes (size as measured in Microsoft Windows®), contains 4 sequences SEQ ID NO: 1 through SEQ ID NO: 4, is filed herewith by electronic submission and is incorporated by reference herein.

The following non-limiting Examples describe methods for modifying the genome of a cotton plant cell using a DSBI enzyme and both Agrobacterium-mediated as well as direct delivery methods on embryogenic callus.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### EXAMPLES

### Example 1: vector construction

Using standard recombinant DNA techniques, the following DNA vectors were constructed comprising the following operably linked elements (schematically depicted in figures 1 and 2):

Target DNA vector pTCV117 (SEQ ID NO 1):
- LB: Left T-DNA border (nt 12540-12564)
- I-Scel site (nt 31-14)
- 3'nos: sequence including the 3' untranslated region of the nopaline synthase gene from the T-DNA of pTiT37 (Depicker *et al.,* 1982) (nt 32-220, reverse complement)
- Bar: coding region of the BAR gene of Streptomyces hygroscopicus (nt 240-791, reverse complement)
- 5'cab22L: sequence including the leader sequence of the chlorophyl a/b binding protein gene from *Petunia hybrida* (Harpster *et al.,* 1988) (nt 801-857, reverse complement)
- P35S2: 35S promoter (nt 858-1405, reverse complement)
- 3'g7: 3' end termination and polyadenylation region of gene 7 of Agrobacterium tumefaciens octopine type T-DNA (nt 1612-1409, reverse complement)
- I-Scel recognition site (nt 1643 - 1617)
- intron1 h3At: sequence including the first intron of gene II of the histone H3.III variant of *Arabidopsis thaliana* (Chaubet *et al*., 1992) (nt 1662-2127)
- TPotpC : coding sequence of the optimized transit peptide, containing sequence of the RuBisCO small subunit genes of *Zea mays* (corn) and *Helianthus annuus* (sunflower), as described by Lebrun *et al.* (1996), (nt 2145-2510)
- 2mepsps: double mutant EPSPS coding region from corn (nt 2517-3852)
- 3'histonAt: sequence including the 3' untranslated region of the histone H4 gene of *Arabidopsis thaliana* (Chabouté *et al*., 1987) (nt 3871-4537)
- RB: right T-DNA border (nt 4594-4618)

Repair DNA vector pTIB232 (SED ID NO 2):
- LB: Left T-DNA border (nt 25-1)
- bar(del1-403): 5'fragment of the BAR gene of Streptomyces hygroscopicus (nt 46-448, reverse complement)
- 5'cab22L: sequence including the leader sequence of the chlorophyl a/b binding protein gene from *Petunia hybrida* (Harpster *et al.,* 1988) (nt 458-514, reverse complement)
- P35S2: 35S promoter (nt 515-1062, reverse complement)
- 3'g7: 3' end termination and polyadenylation region of gene 7 of Agrobacterium tumefaciens octopine type T-DNA (nt1409-1612)
- Pcsvmv XYZ: CsVMV promoter fragment (1285-1724)
- 5'csvmv: leader of CsVMV promoter (nt 1725-1797)
- hyg-1 Pa: Hygromycin resistance gene of E.coli (nt 1804-2829)
- 3'35S: 3' 35S transcription termination and polyadenylation region (nt 2841-3065)
- 2mepsps(5'del): 3' fragment of the double mutant EPSPS gene from corn (nt 3096-3501)
- 3'histonAt: 3' end region of the histon 4 gene from Arabidopsis (nt 3520-4186)
- RB: Right T-DNA border (nt 4267-4243)

Repair DNA and endonuclease expression vector pTIB236 (SEQ ID NO 3):
- LB: Left T-DNA border (nt 1-25)
- 3'35S: 35S transcription termination and polyadenylation region (nt 104-328, reverse complement)
- hyg-1 Pa: hygromycine resistance gene from E. coli (nt 340-1365, reverse complement)
- 5'csvmv: leader of CsVMV (nt 1372-1444, reverse complement)
- Pcsvmv XYZ: CsVMV promoter fragment (1445-1884, reverse complement)
- 3'35S: 35S polyadenylation region (nt 1963-2097, reverse complement)
- I-Scel: universal code I-Scel coding region (nt 2185-2919, reverse complement)
- NLSsv40: SV40 nuclear localization signal (nt-2887-2910-, reverse complement)
- 5'ats1b: leader sequence from *Arabidopsis thaliana* rbcS ATS1A gene (nt 2936-2976, reverse complement)
- P35S2: 35S promoter (nt 2976-3496, reverse complement)
- RB: Right T-DNA border (nt 4592-5655)

Endonuclease expression vector ptrr26 (SEQ ID NO 4) comprising the universal code I-Scel coding region (WO 2006/074956):
- RB: Right T-DNA border
- P35S2: 35S promoter
- 5'ats1b: leader sequence from *Arabidopsis thaliana* rbcS ATS1A gene
- NLSsv40: SV40 nuclear localization signal
- I-Scel: universal code I-Scel coding region
- 3'35S
- LB: Left T-DNA border

### Example 2: Media and buffers

Media and buffers used during the embryonic callus generation and transformation as described below in examples 3, 4 and 5:
**Co-cultivation substrate:** M100 with ½ concentration MS salts pH=5.2, + 100 µM AS + 100 mg/L L-cysteïne (L-cysteïne has always to be freshly prepared and added after autoclavation)
**M100 substrate:** MS salts, B5 vitamins, MES 0.5 g/L, MgCl₂.6H₂O 0.94 g/L, gelrite 2g/L, glucose 30 g/L, pH 5.8
**M104 substrate:** = M100 substrate + 1 g/L KNO₃, pH 5.8
**M700 substrate:** Stewarts salts + vitamins, MgCl₂.6H₂O 0.47 g/L, gelrite 1g/L, plant agar 2.25 g/L, sucrose 20 g/L, pH 6.8
**M702 substrate:** Stewarts salts + vitamins, MgCl₂.6H₂O 0.71 g/L, gelrite 1.5 g/L, plant agar 5 g/L, sucrose 5 g/L, pH 6.8 **AC:** active carbon 2g/L
**AS:** acetosyringone 100µM in DMSO

### Example 3: Generation of friable embryogenic callus

Cotton seeds from Coker 312 were germinated on solid germination medium M100 without hormones for 7-10 days in the dark at 28°C. Next, induction of embryogenic callus was performed by incubating hypocotyl explants from the seedlings on solid M100 medium (without hormones). After about 2 months when the wound callus at the cut surface of the hypocotyls starts to show fast proliferation, the further subculture for enrichment and maintenance of embryogenic callus is done on solid M100 medium with active carbon (2g/L). Induction and maintenance of embryogenic callus occurs under dim light conditions (intensity: 1 to 7 µmol m⁻² sec⁻¹; photoperiod: 16H light/8H dark) at 28°C.

### Example 4: Transformation of cotton embryogenic callus by particle bombardment

The following procedure was followed to transform cotton embryogenic callus using particle bombardment:
- Friable cotton embryogenic callus (EC) of example 3 of a target line in which to introduce a DSB induced targeted modification, is collected 2 to 3 weeks after subculture and plated in a thin layer by means of a Büchnerfilter on top of a filter paper on M100 substrate with 0.2M mannitol and 0.2M sorbitol for ∼2 to ∼20 hours prior to bombardment.
- After preplasmolysis on M100 substrate with 0.2M mannitol and 0.2M sorbitol for ∼2 to ∼20 hours, the EC is bombarded with the endonuclease DNA (∼0.5 pmol) optionally in the presence of a repair DNA (∼0.5 pmol)
- Bombardment conditions:
   ∘ diameter gold particles: 0.3-3µm
   ∘ rupture disc: 1100-1350 psi
   ∘ distance to target tissue: 9 cm
   ∘ chamber vacuum ∼27 (in Hg)
   ∘ BioRAD PPS_1000/He Biolistic Particle delivering system
- After bombardment, the filters are transferred onto M100 substrate with 0.2 M mannitol or M100 substrate without selective agent.
- After 1 to 4 days on non-selective substrate under dimlight conditions at 28°C, the filters are transferred onto selective M100 substrate with either 50 mg/L hygromycin or 1 mM glyphosate or 5 mg/L PPT
- After about 2 to 3 weeks, proliferating calli are selected from the filters and further subcultured as small piles onto selective M100 substrate with either 50 mg/L hygromycin or 1 mM glyphosate or 5 mg/L PPT. After a subculture period of ∼6 weeks with ∼3 weekly subculture intervals, on selective M100 substrate under dimlight conditions at 28°C, transformed EC/somatic embryos can be selected.
- A molecular screen for the identification of targeted modification events is performed at the level of transformed EC/somatic embryos.
- Plant regeneration is initiated from the targeted modification events by plating EC/somatic embryos on M104 with active carbon (AC) and the corresponding selective agent under light conditions (intensity: 40 to 70 µmol m-²sec⁻¹; photoperiod: 16H light/8H dark) at 28°C.
- After about one month individual embryos of about 0.5-1cm are transferred on top of a filter paper on M104 with AC and the corresponding selective agent.
- Further well germinating embryos are transferred onto non-selective germination substrate M702 under light conditions (intensity: 40 to 70 µmol m⁻² spec⁻¹; photoperiod :16H light/8H dark) at 28°C.
- After one to two months the further developing embryos are transferred onto M700 substrate under light conditions (intensity: 40 to 70 µmol m⁻² sec⁻¹; photoperiod :16H light/8H dark) at 28°C for development into small plantlets.

### Example 5: Transformation of cotton embryogenic callus by Agrobacterium-mediated DNA delivery

The following procedure was followed to transform cotton embryogenic callus using Agrobacterium:
- Friable cotton embryogenic callus (EC) of example 3 of a target line in which to to introduce a DSB induced targeted modification is collected 2 to 3 weeks after subculture on substrate 100 and immersed for 20' in an Agrobacterium suspension of 5x10⁸ cells/ml in M100 substrate pH 5.2, with 100 µM acetosyringone (AS). The Agrobacterium strain carries a vector containing the repair DNA and the gene encoding the endonuclease.
- After 3 days co-cultivation in the dark at 24°C on M100 with ½ concentration MS salts pH 5.2, with100 µM AS and 100 mg/L L-cysteïne, the EC is either plated on top of a filter paper by means of a Büchner filter or transferred as small piles on M100 substrate pH5.8, 250mg/L triacillin and a selective agent (hygromycin 50mg/L or PPT 5 mg/L or glyphosate 1 to 1.5 mM) and incubated in dim light (intensity: 1 to 7 µmol m⁻² sec⁻¹; 16H light/8H dark) at 28°C.
- After 2 to 3 weeks, calli are further subcultured as small piles onto selective M100 substrate with either 50 mg/L hygromycin or 1 mM glyphosate or 5 mg/L PPT. After a subculture period for ∼6 weeks with ∼3 weekly subculture intervals, on selective M100 substrate under dimlight conditions at 28°C, transformed EC/somatic embryos can be selected.
- A molecular screen for the identification of targeted modification events is performed at the level of transformed EC/somatic embryos.
- Plant regeneration is initiated from the targeted modification events by plating EC/somatic embryos on M104 with active carbon and the corresponding selective agent under light conditions (intensity: 40 to 70 µmol m⁻² sec⁻¹; photoperiod 16H light/8H dark) at 28°C. From this step on, the substrates do not contain anymore antibiotics.
- After about one month individual embryos of about 0.5-1cm are transferred on top of a filter paper on M104 with active carbon (AC) and the corresponding selective substrate.
- Further germinating embryos are transferred on non-selective germination substrate M702 under light conditions (intensity: 40 to 70 µmol m⁻² sec⁻¹; photoperiod (16H light/8H dark at 28°C.
- After one to two months the further developing embryos are transferred onto M700 substrate under light conditions (intensity: 40 to 70 µmol m⁻² sec⁻¹; photoperiod (16H light/8H dark) at 28°C for development into small plantlets.

### Example 6: Generation of target plants for the evaluation of targeted genome engineering

Transgenic cotton plants comprising the pTCV117 target DNA vector were generated by Agrobacterium as described in example 5. The pTCV117 vector comprises a functional 35S-driven bar gene located between two I-Scel recognition sites and a promoterless EPSPS gene (see figure 1). To evaluate targeted recombination, it was originally intended to transform these plants with a 35S-I-Scel expression cassette and a repair DNA with homology regions to the target DNA for restoration of the promoter-less epsps gene by insertion of a histon promoter, thereby resulting in the acquisition of glyphosate tolerance. However, these pTCV117 plants appeared to already have high levels of tolerance to glyphosate, probably due to bidirectional transcriptional activity of the 35S promoter, thereby making the assay unusable.

### Example 7: Targeted insertion or replacement through homologous recombination

Thus, a new repair DNA pTIB232 with homology regions to the target DNA was constructed, comprising a functional CSVMV-driven hygromycin resistance gene flanked at one end by a 3' epsps gene fragment (allowing homologous recombination with the EPSPS gene at the target locus, thereby replacing the 5' part of the EPSPS gene by the hygromycin gene) and at the other end flanked by a 35S-promoter linked to a 5' bar gene fragment), as schematically indicated in figure 1. pTCV117 target plants were transformed with the pTIB232 vector and the ptrr26 vector comprising a universal code I-Scel coding region operably linked to a 35S promoter using particle bombardment as described in example 4.

First, transformants were screened for hygromycin tolerance, as this indicates insertion of the repair DNA pTIB232. HygR events were subsequently evaluated for loss of glyphosate resistance, which is indicative of recombination at the target locus. The 36 thus obtained hygR and GlyS recombinants were further characterized by PCR analysis using primer pair P3 xP4 recognizing the genomic region upstream of the EPSPS gene and the hygromycin gene (Figure 1),. This resulted in the identification of 8 potential correct gene targeting events (replacement of the 5' part of the epsps gene by the hygromycin gene by at least one-sided homologous recombination: configuration I in figure 1), which were subsequently confirmed to be indeed correct gene targeting events by sequence analysis of the PCR product obtained by the primer set P3, P4).

### Example 8: Targeted insertion or replacement through non-homologous end-joining

pTCV117 plants were transformed with repair DNA and endonuclease expression vector pTIB236, comprising a functional hygR gene and a functional universal code I-Scel encoding gene, with no homology to the target site (see figure 2), using Agrobacteruim-mediated DNA transfer as described in example 5.

The 200 hygR events that were thus obtained, were analyzed by PCR using 2 primer pairs P1xP2 and P3xP4; the primer pair P1xP2 recognizing the hygR gene and the genomic region downstream of the bar gene and the other pair recognizing the genomic region upstream of the EPSPS gene and the hygromycin gene (Figure 2). This resulted in the identification of 17 potential targeted insertion/replacement events. Sequence analysis done on 4 of these events showed that they were indeed targeted insertion / replacement events (configuration I , II in Figure 2).

### Example 9: Targeted insertion or replacement via non-homologous end joining using Agrobacterium-mediated transformation

Target line G4GH198-01901 was transformed as described in Example 5 with Agrobacterium strain A5280=EHA101(pTIB236) comprising the repair DNA and endonuclease expression vector pTIB236.

Hygromycin resistant (HygR) events were selected of which 200 were analyzed by PCR for insertion of the hyg gene at the target I-Scel site(s) using primer pairs P1xP2 and P3xP4. Of these 200 events, about 10% were found to be targeted events. Sequence analysis of some of these targeted events revealed that either the bar gene had been replaced by the hyg gene or that the hyg gene had inserted next to the bar gene (stacked event).

### Example 10: Targeted double-stranded break induction using a custom designed meganuclease

Embryogenic calli from PPT-resistant cotton plants containing a chimeric gene comprising the bar gene under control of the CSVMV promoter were transformed via particle bombardment with a vector comprising a chimeric gene encoding a custom engineered meganuclease recognizing a target sequence in the bar gene, either as a single chain (pCV170: SEQ ID NO. 5) or as a heterodimer (pCV177: SEQ ID NO. 6). A co-delivery was done of the meganuclease vector with a vector containing the 2mEPSPS gene under control of a plant-expressible promoter conferring glyphosate tolerance as a selectable marker gene. About 3000 glyphosate resistant calli were obtained of which 85 events appeared PPT sensitive, indicating a disruption of the bar gene. Of these, 79 events were characterized for genotype by PCR using primers flanking the target site and subsequent sequencing of the PCR product (table 1).

**Table 1: Characterization of PPT-sensitive glyphosate-resistant transformation events. The absence of a PCR product is indicative of a large deletion around the target site.**

| **pCV170 (sc)** | | | |
|---|---|---|---|
| PCR product obtainable | # events | change at target site | # events |
| no | 11 | large deletion | 11 |
| yes | 8 | no mutation | 6 |
| | | replacement/insertion | 1 |
| | | deletion | 1 |

| **pCV177 (hd)** | | | |
|---|---|---|---|
| PCR product obtainable | # events | change at target site | # events |
| no | 33 | large deletion | 33 |
| yes | 27 | no mutation | 19 |
| | | insertion | 4 |
| | | deletion | 4 |

Thus, these results demonstrate that it is possible to induce a targeted double stranded DNA break at the desired position with both a single chain as well as a heterodimeric custom-designed meganuclease and that targeted deletion, replacement and insertion events can be obtained using such meganucleases in cotton.

## Claims

1. A method for modifying the genome of a cotton plant cell at a predefined site comprising the steps of
a. inducing a double stranded DNA break in the vicinity of or at said predefined site, said double stranded break being induced by the introduction into said cell of a rare-cleaving endonuclease enzyme which recognizes a recognition sequence in the vicinity of or at said predefined site;
b. selecting a plant cell wherein said double stranded DNA break has been repaired resulting in a modification in the genome at said preselected site, wherein said modification is selected from
i. a replacement of at least one nucleotide;
ii. a deletion of at least one nucleotide;
iii. an insertion of at least one nucleotide; or
iv. any combination of i. - iii.;
**characterized in that** said cell is comprised within friable embryogenic callus, wherein said friable embryogenic callus has been maintained under dim light conditions and on medium comprising active carbon, wherein said dim light conditions involve an intensity of about 1 to 7 µmol m-2 sec-1 with a photoperiod of about 16 hours light/8 hours dark..

2. The method of claim 1, wherein said friable embryogenic callus has been maintained under dim light conditions and on medium comprising active carbon for about 2 to 4 months.

3. The method of claim 1 or 2, wherein said endonuclease enzyme is introduced into said cell by the delivery into said cell of a DNA molecule encoding said endonuclease enzyme.

4. The method of any one of claims 1-3, wherein prior to step b. a foreign repair DNA molecule is delivered into said cell, said foreign repair DNA molecule being used as a template for repair of said double stranded DNA break.

5. The method of any one of claims 1-4, wherein said embryogenic callus is incubated in medium without hormones.

6. The method of any one of claims 1-5, wherein said embryogenic callus is incubated for 1 to 4 days on a non-selective medium during or after said introduction of said endonuclease enzyme.

7. The method of any one of claims 3-6, wherein said DNA delivery is performed by particle bombardment or by using Agrobacterium.

8. The method according to any one of claims 3-7, wherein said foreign repair DNA comprises at least one flanking nucleotide sequence having sufficient homology to the upstream or downstream DNA region of said predefined site to allow recombination with said upstream or downstream DNA region or wherein said foreign repair DNA comprises two flanking nucleotide sequences located on opposite ends of said foreign DNA, one of said flanking nucleotide sequence having sufficient homology to the upstream DNA region of said predefined site, the other flanking nucleotide sequence having sufficient homology to the downstream sequence of said predefined site to allow recombination between said flanking nucleotide sequences and said upstream and downstream DNA regions.

9. The method according to any one of claims 4-8, wherein said foreign repair DNA comprises a selectable marker gene.

10. The method according to any one of claims 4-9, wherein said foreign DNA comprises a plant expressible gene of interest.

11. The method according to claim 10, wherein said plant expressible gene of interest is selected from the group of a herbicide tolerance gene, an insect resistance gene, a disease resistance gene, an abiotic stress resistance gene,
a gene encoding an enzyme involved in oil biosynthesis or carbohydrate biosynthesis, a gene encoding an enzyme involved in fiber strength or fiber length, a gene encoding an enzyme involved in biosynthesis of secondary metabolites.

12. The method according to any one of claims 4-7, wherein said foreign DNA consists of two flanking nucleotide sequences, one of said flanking nucleotide sequence having sufficient homology to the upstream DNA region of said predefined site, the other flanking nucleotide sequence having sufficient homology to the downstream sequence of said predefined site to allow recombination between said flanking nucleotide sequences and said upstream and downstream DNA regions.

13. The method of any one of claims 1-3 or 5-7, wherein said preselected site is flanked by two regions with sufficient homology for recombination with each other.

14. The method according to any one of claims 1 to 13, wherein said cotton plant cell is further regenerated into a cotton plant.

## Patentansprüche

1. Verfahren zum Modifizieren des Genoms einer Baumwollpflanzenzelle an einer vordefinierten Stelle, umfassend die folgenden Schritte:
a. Induzieren eines Doppelstrang-DNA-Bruchs in der Nähe oder an der vordefinierten Stelle, wobei der Doppelstrangbruch dadurch induziert wird, dass man eine selten spaltende Endonuklease, die eine Erkennungssequenz in der Nähe oder an der vordefinierten Stelle erkennt, in die Zelle einführt;
b. Selektieren einer Pflanzenzelle, in der der Doppelstrang-DNA-Bruch so repariert worden ist, dass das Ergebnis eine Modifikation in dem Genom an der vorgewählten Stelle ist, wobei die Modifikation aus den Folgenden ausgewählt ist:
i. einem Ersatz von mindestens einem Nukleotid;
ii. einer Deletion von mindestens einem Nukleotid;
iii. einer Insertion von mindestens einem Nukleotid; oder
iv. einer beliebigen Kombination von i. - iii.;
**dadurch gekennzeichnet, dass** die Zelle innerhalb von lockerem embryogenem Kallus umfasst ist, wobei der lockere embryogene Kallus unter Schwachlichtbedingungen und auf Medium, das Aktivkohle umfasst, aufrechterhalten wurde, wobei die Schwachlichtbedingungen eine Intensität von ungefähr 1 bis 7 µmol m-2 sec-1 mit einer Photoperiode von ungefähr 16 Stunden Licht/8 Stunden Dunkelheit beinhalten.

2. Verfahren nach Anspruch 1, wobei der lockere embryogene Kallus ungefähr 2 bis 4 Monate unter Schwachlichtbedingungen und auf Medium, das Aktivkohle umfasst, aufrechterhalten wurde.

3. Verfahren nach Anspruch 1 oder 2, wobei die Endonuklease in die Zelle durch Abgabe eines DNA-Moleküls, das für die Endonuklease codiert, in die Zelle eingebracht wird.

4. Verfahren nach einem der Ansprüche 1-3, wobei vor Schritt b. ein Fremd-Reparatur-DNA-Molekül in die Zelle abgegeben wird, wobei das Fremd-Reparatur-DNA-Molekül als Matrize für die Reparatur des Doppelstrang-DNA-Bruchs verwendet wird.

5. Verfahren nach einem der Ansprüche 1-4, wobei der embryogene Kallus in Medium ohne Hormone inkubiert wird.

6. Verfahren nach einem der Ansprüche 1-5, wobei der embryogene Kallus während oder nach der Einbringung der Endonuklease 1 bis 4 Tage auf einem nichtselektiven Medium inkubiert wird.

7. Verfahren nach einem der Ansprüche 3-6, wobei die DNA-Abgabe mit der Genkanone oder durch Einsatz von Agrobakterium erfolgt.

8. Verfahren nach einem der Ansprüche 3-7, wobei die Fremd-Reparatur-DNA mindestens eine flankierende Nukleotidsequenz mit ausreichend Homologie zu der stromaufwärts oder stromabwärts gelegenen DNA-Region der vordefinierten Stelle umfasst, um eine Rekombination mit der stromaufwärts oder stromabwärts gelegenen DNA-Region zu ermöglichen, oder wobei die Fremd-Reparatur-DNA zwei flankierende Nukleotidsequenzen umfasst, die an gegenüberliegenden Enden der Fremd-DNA liegen, wobei eine der flankierenden Nukleotidsequenzen ausreichend Homologie mit der stromaufwärts gelegenen DNA-Region der vordefinierten Stelle aufweist und die andere flankierende Nukleotidsequenz ausreichend Homologie mit der stromabwärts gelegenen Sequenz der vordefinierten Stelle aufweist, um eine Rekombination zwischen den flankierenden Nukleotidsequenzen und den stromaufwärts und stromabwärts gelegenen DNA-Regionen zu gestatten.

9. Verfahren nach einem der Ansprüche 4-8, wobei die Fremd-Reparatur-DNA ein Selektionsmarkergen umfasst.

10. Verfahren nach einem der Ansprüche 4-9, wobei die Fremd-DNA ein in Pflanzen exprimierbares Gen von Interesse umfasst.

11. Verfahren nach Anspruch 10, wobei das in Pflanzen exprimierbare Gen von Interesse aus der Gruppe Herbizidtoleranzgen, Insektenresistenzgen, Krankheitsresistenzgen, Gen für Resistenz gegen abiotischen Stress, Gen das für ein an der Ölbiosynthese oder Kohlenhydratbiosynthese beteiligten Enzym kodiert, Gen das für ein an Faserstärke oder Faserlänge beteiligten Enzym kodiert, Gen das für ein an der Biosynthese von Sekundärmetaboliten beteiligten Enzym kodiert, ausgewählt ist.

12. Verfahren nach einem der Ansprüche 4-7, wobei die Fremd-DNA aus zwei flankierenden Nukleotidsequenzen besteht, wobei eine der flankierenden Nukleotidsequenzen ausreichend Homologie mit der stromaufwärts gelegenen DNA-Region der vordefinierten Stelle aufweist und die andere flankierende Nukleotidsequenz ausreichend Homologie mit der stromabwärts gelegenen Sequenz der vordefinierten Stelle aufweist, um eine Rekombination zwischen den flankierenden Nukleotidsequenzen und den stromaufwärts und stromabwärts gelegenen DNA-Regionen zu gestatten.

13. Verfahren nach einem der Ansprüche 1-3 oder 5-7, wobei die vorgewählte Stelle von zwei Regionen mit ausreichend Homologie für eine Rekombination miteinander flankiert wird.

14. Verfahren nach einem der Ansprüche 1-13, wobei die Baumwollpflanzenzelle zu einer Baumwollpflanze weiter regeneriert wird.

## Revendications

1. Procédé de modification du génome d'une cellule de plante de coton à un site prédéfini comprenant les étapes de
a. induction d'une rupture d'ADN double brin à proximité dudit ou audit site prédéfini, ladite rupture double brin étant induite par l'introduction dans ladite cellule d'une enzyme endonucléase à clivage rare qui reconnaît une séquence de reconnaissance à proximité dudit ou audit site prédéfini ;
b. sélection d'une cellule de plante dans laquelle ladite rupture d'ADN double brin a été réparée de manière à conduire à une modification dans le génome audit site présélectionné, ladite modification étant choisie parmi
i. un remplacement d'au moins un nucléotide;
ii. une délétion d'au moins un nucléotide ;
iii. une insertion d'au moins un nucléotide; ou
iv. une combinaison quelconque de i. à iii.;
**caractérisé en ce que** ladite cellule est comprise dans un cal embryogène friable, ledit cal embryogène friable ayant été maintenu dans des conditions de lumière réduite et sur un milieu comprenant du charbon actif, lesdites conditions de lumière réduite mettant en oeuvre une intensité d'environ 1 à 7 µmol.m-2.s-1 avec une photopériode d'environ 16 heures de lumière/8 heures d'obscurité.

2. Procédé de la revendication 1, dans lequel ledit cal embryogène friable a été maintenu dans des conditions de lumière réduite et sur un milieu comprenant du charbon actif pendant environ 2 à 4 mois.

3. Procédé de la revendication 1 ou 2, dans lequel ladite enzyme endonucléase est introduite dans ladite cellule par l'administration dans ladite cellule d'une molécule d'ADN codant pour ladite enzyme endonucléase.

4. Procédé de l'une quelconque des revendications 1 à 3, dans lequel, avant l'étape b., une molécule d'ADN de réparation étrangère est administrée dans ladite cellule, ladite molécule d'ADN de réparation étrangère étant utilisée en tant que matrice pour la réparation de ladite rupture d'ADN double brin.

5. Procédé de l'une quelconque des revendications 1 à 4, dans lequel ledit cal embryogène est incubé dans du milieu sans hormones.

6. Procédé de l'une quelconque des revendications 1 à 5, dans lequel ledit cal embryogène est incubé pendant 1 à 4 jours sur un milieu non sélectif pendant ou après ladite introduction de ladite enzyme endonucléase.

7. Procédé de l'une quelconque des revendications 3 à 6, dans lequel ladite administration d'ADN est effectuée par bombardement de particules ou en utilisant Agrobacterium.

8. Procédé selon l'une quelconque des revendications 3 à 7, dans lequel ledit ADN de réparation étranger comprend au moins une séquence nucléotidique flanquante ayant une homologie suffisante avec la région d'ADN en amont ou en aval dudit site prédéfini pour permettre une recombinaison avec ladite région d'ADN en amont ou en aval ou dans lequel ledit ADN de réparation étranger comprend deux séquences nucléotidiques flanquantes situées sur des extrémités opposées dudit ADN étranger, l'une desdites séquences nucléotidiques flanquantes ayant une homologie suffisante avec la région d'ADN en amont dudit site prédéfini, l'autre séquence nucléotidique flanquante ayant une homologie suffisante avec la séquence en aval dudit site prédéfini pour permettre une recombinaison entre lesdites séquences nucléotidiques flanquantes et lesdites régions d'ADN en amont et en aval.

9. Procédé selon l'une quelconque des revendications 4 à 8, dans lequel ledit ADN de réparation étranger comprend un gène marqueur sélectionnable.

10. Procédé selon l'une quelconque des revendications 4 à 9, dans lequel ledit ADN étranger comprend un gène de plante d'intérêt exprimable.

11. Procédé selon la revendication 10, dans lequel ledit gène de plante d'intérêt exprimable est choisi dans le groupe d'un gène de tolérance aux herbicides, un gène de résistance aux insectes, un gène de résistance aux maladies, un gène de résistance au stress abiotique, un gène codant pour une enzyme impliquée dans la biosynthèse d'huiles ou la biosynthèse de glucides, un gène codant pour une enzyme impliquée dans la résistance de fibres ou la longueur de fibres, un gène codant pour une enzyme impliquée dans la biosynthèse de métabolites secondaires.

12. Procédé selon l'une quelconque des revendications 4 à 7, dans lequel ledit ADN étranger est constitué de deux séquences nucléotidiques flanquantes, l'une desdites séquences nucléotidiques flanquantes ayant une homologie suffisante avec la région d'ADN en amont dudit site prédéfini, l'autre séquence nucléotidique flanquante ayant une homologie suffisante avec la séquence en aval dudit site prédéfini pour permettre une recombinaison entre lesdites séquences nucléotidiques flanquantes et lesdites régions d'ADN en amont et en aval.

13. Procédé de l'une quelconque des revendications 1 à 3 ou 5 à 7, dans lequel ledit site présélectionné est flanqué par deux régions ayant une homologie suffisante pour recombinaison l'une avec l'autre.

14. Procédé selon l'une quelconque des revendications 1 à 13, dans lequel ladite cellule de plante de coton est ensuite régénérée en plante de coton.


### SEQUENCE LISTING

<110> Bayer CropScience N.V. D'Halluin, Katelijn
<120> METHODS AND MEANS TO MODIFY A PLANT GENOME AT A PRESELECTED SITE
<130> BCS11-2008
<160> 4
<170> PatentIn version 3.3
<210> 1
   <211> 12641
   <212> DNA
   <213> Artificial
<220>
   <223> vector
<400> 1
   agcttgcgat cgctagggat aacagggtaa tcgcgtatta aatgtataat tgcgggactc      60
   taatcataaa aacccatctc ataaataacg tcatgcatta catgttaatt attacatgct     120
   taacgtaatt caacagaaat tatatgataa tcatcgcaag accggcaaca ggattcaatc     180
   ttaagaaact ttattgccaa atgtttgaac gatctgcttc ggatcctaga cgcgtgagat     240
   cagatctcgg tgacgggcag gaccggacgg ggcggtaccg gcaggctgaa gtccagctgc     300
   cagaaaccca cgtcatgcca gttcccgtgc ttgaagccgg ccgcccgcag catgccgcgg     360
   ggggcatatc cgagcgcctc gtgcatgcgc acgctcgggt cgttgggcag cccgatgaca     420
   gcgaccacgc tcttgaagcc ctgtgcctcc agggacttca gcaggtgggt gtagagcgtg     480
   gagcccagtc ccgtccgctg gtggcggggg gagacgtaca cggtcgactc ggccgtccag     540
   tcgtaggcgt tgcgtgcctt ccaggggccc gcgtaggcga tgccggcgac ctcgccgtcc     600
   acctcggcga cgagccaggg atagcgctcc cgcagacgga cgaggtcgtc cgtccactcc     660
   tgcggttcct gcggctcggt acggaagttg accgtgcttg tctcgatgta gtggttgacg     720
   atggtgcaga ccgccggcat gtccgcctcg gtggcacggc ggatgtcggc cgggcgtcgt     780
   tctgggtcca tggttttggt ttaataagaa gagaaaagag ttcttttgtt atggctgaag     840
   taatagagaa atgagctcga gtcctctcca aatgaaatga acttccttat atagaggaag     900
   ggtcttgcga aggatagtgg gattgtgcgt catcccttac gtcagtggag atatcacatc     960
   aatccacttg ctttgaagac gtggttggaa cgtcttcttt ttccacgatg ctcctcgtgg    1020
   gtgggggtcc atctttggga ccactgtcgg cagaggcatc ttgaacgata gcctttcctt    1080
   tatcgcaatg atggcatttg taggtgccac cttccttttc tactgtcctt ttgatgaagt    1140
   gacagatagc tgggcaatgg aatccgagga ggtttcccga tattaccctt tgttgaaaag    1200
   tctcaatagc cctttggtct tctgagactg tatctttgat attcttggag tagacgagag    1260
   tgtcgtgctc caccatgttg acgaagattt tcttcttgtc attgagtcgt aaaagactct 1320
   gtatgaactg ttcgccagtc ttcacggcga gttctgttag atcctcgatc tgaatttttg 1380
   actccatgta tggtgcatat ggcgcgccta agctagctag atcatcaatt tatgtattac 1440
   acataatatc gcactcagtc tttcatctac ggcaatgtac cagctgatat aatcagttat 1500
   tgaaatattt ctgaatttaa acttgcatca ataaatttat gtttttgctt ggactataat 1560
   acctgacttg ttattttatc aataaatatt taaactatat ttctttcaag atacgtatta 1620
   ccctgttatc cctaaagctt atcgatttcg aacccctcag gcgaagaaca ggtatgattt 1680
   gtttgtaatt agatcagggg tttaggtctt tccattactt tttaatgttt tttctgttac 1740
   tgtctccgcg atctgatttt acgacaatag agtttcgggt tttgtcccat tccagtttga 1800
   aaataaaggt ccgtctttta agtttgctgg atcgataaac ctgtgaagat tgagtctagt 1860
   cgatttattg gatgatccat tcttcatcgt ttttttcttg cttcgaagtt ctgtataacc 1920
   agatttgtct gtgtgcgatt gtcattacct agccgtgtat cgagaactag ggttttcgag 1980
   tcaattttgc cccttttggt tatatctggt tcgataacga ttcatctgga ttagggtttt 2040
   aagtggtgac gtttagtatt ccaatttctt caaaatttag ttatggataa tgaaaatccc 2100
   caattgactg ttcaatttct tgttaaatgc gcagatcccc atggcttcga tctcctcctc 2160
   agtcgcgacc gttagccgga ccgcccctgc tcaggccaac atggtggctc cgttcaccgg 2220
   ccttaagtcc aacgccgcct tccccaccac caagaaggct aacgacttct ccacccttcc 2280
   cagcaacggt ggaagagttc aatgtatgca ggtgtggccg gcctacggca acaagaagtt 2340
   cgagacgctg tcgtacctgc cgccgctgtc tatggcgccc accgtgatga tggcctcgtc 2400
   ggccaccgcc gtcgctccgt tccaggggct caagtccacc gccagcctcc ccgtcgcccg 2460
   ccgctcctcc agaagcctcg gcaacgtcag caacggcgga aggatccggt gcatggccgg 2520
   cgccgaggag atcgtgctgc agcccatcaa ggagatctcc ggcaccgtca agctgccggg 2580
   gtccaagtcg ctttccaacc ggatcctcct actcgccgcc ctgtccgagg ggacaacagt 2640
   ggttgataac ctgctgaaca gtgaggatgt ccactacatg ctcggggcct tgaggactct 2700
   tggtctctct gtcgaagcgg acaaagctgc caaaagagct gtagttgttg gctgtggtgg 2760
   aaagttccca gttgaggatg ctaaagagga agtgcagctc ttcttgggga atgctggaat 2820
   cgcaatgcgg tccttgacag cagctgttac tgctgctggt ggaaatgcaa cttacgtgct 2880
   tgatggagta ccaagaatga gggagagacc cattggcgac ttggttgtcg gattgaagca 2940
   gcttggtgca gatgttgatt gtttccttgg cactgactgc ccacctgttc gtgtcaatgg 3000
   aatcggaggg ctacctggtg gcaaggtcaa gctgtctggc tccatcagca gtcagtactt 3060
   gagtgccttg ctgatggctg ctcctttggc tcttggggat gtggagattg aaatcattga 3120
   taaattaatc tccattccgt acgtcgaaat gacattgaga ttgatggagc gttttggtgt 3180
   gaaagcagag cattctgata gctgggacag attctacatt aagggaggtc aaaaatacaa 3240
   gtcccctaaa aatgcctatg ttgaaggtga tgcctcaagc gcaagctatt tcttggctgg 3300
   tgctgcaatt actggaggga ctgtgactgt ggaaggttgt ggcaccacca gtttgcaggg 3360
   tgatgtgaag tttgctgagg tactggagat gatgggagcg aaggttacat ggaccgagac 3420
   tagcgtaact gttactggcc caccgcggga gccatttggg aggaaacacc tcaaggcgat 3480
   tgatgtcaac atgaacaaga tgcctgatgt cgccatgact cttgctgtgg ttgccctctt 3540
   tgccgatggc ccgacagcca tcagagacgt ggcttcctgg agagtaaagg agaccgagag 3600
   gatggttgcg atccggacgg agctaaccaa gctgggagca tctgttgagg aagggccgga 3660
   ctactgcatc atcacgccgc cggagaagct gaacgtgacg gcgatcgaca cgtacgacga 3720
   ccacaggatg gcgatggctt tctcccttgc cgcctgtgcc gaggtccccg tcaccatccg 3780
   ggaccctggg tgcacccgga agaccttccc cgactacttc gatgtgctga gcactttcgt 3840
   caagaattaa gctctagaac tagtggatcc cccgatccgc gtttgtgttt tctgggtttc 3900
   tcacttaagc gtctgcgttt tacttttgta ttgggtttgg cgtttagtag tttgcggtag 3960
   cgttcttgtt atgtgtaatt acgctttttc ttcttgcttc agcagtttcg gttgaaatat 4020
   aaatcgaatc aagtttcact ttatcagcgt tgttttaaat tttggcatta aattggtgaa 4080
   aattgcttca attttgtatc taaatagaag agacaacatg aaattcgact tttgacctca 4140
   aatcttcgaa catttatttc ctgatttcac gatggatgag gataacgaaa gggcggttcc 4200
   tatgtccggg aaagttcccg tagaagacaa tgagcaaagc tactgaaacg cggacacgac 4260
   gtcgcattgg tacggatatg agttaaaccg actcaattcc tttattaaga cataaaccga 4320
   ttttggttaa agtgtaacag tgagctgata taaaaccgaa acaaaccggt acaagtttga 4380
   ttgagcaact tgatgacaaa cttcagaatt ttggttattg aatgaaaatc atagtctaat 4440
   cgtaaaaaat gtacagaaga aaagctagag cagaacaaag attctatatt ctggttccaa 4500
   tttatcatcg ctttaacgtc cctcagattt gatcgggctg caggaattaa acgcccgggc 4560
   acgtgggatc ctctagagtc gacggccgag tactggcagg atatataccg ttgtaatttg 4620
   tcgcgtgtga ataagtcgct gtgtatgttt gtttgattgt ttctgttgga gtgcagccca 4680
   tttcaccgga caagtcggct agattgattt agccctgatg aactgccgag gggaagccat 4740
   cttgagcgcg gaatgggaat ggatttcgtt gtacaacgag acgacagaac acccacggga 4800
   ccgagcttcg atcgagcatc aaatgaaact gcaatttatt catatcagga ttatcaatac 4860
   catatttttg aaaaagccgt ttctgtaatg aaggagaaaa ctcaccgagg cagttccata 4920
   ggatggcaag atcctggtat cggtctgcga ttccgactcg tccaacatca atacaaccta 4980
   ttaatttccc ctcgtcaaaa ataaggttat caagtgagaa atcaccatga gtgacgactg 5040
   aatccggtga gaatggcaaa agtttatgca tttctttcca gacttgttca acaggccagc 5100
   cattacgctc gtcatcaaaa tcactcgcat caaccaaacc gttattcatt cgtgattgcg 5160
   cctgagcgag acgaaatacg ccgctgttaa aaggacaatt acaaacagga atcgaatgca 5220
   accggcgcag gaacactgcc agcgcatcaa caatattttc acctgaatca ggatattctt 5280
   ctaatacctg gaatgctgtt tttccgggga tcgcagtggt gagtaaccat gcatcatcag 5340
   gagtacggat aaaatgcttg atggtcggaa gaggcataaa ttccgtcagc cagtttagtc 5400
   tgaccatctc atctgtaaca tcattggcaa cgctaccttt gccatgtttc agaaacaact 5460
   ctggcgcatc gggcttccca tacaatcgat agattgtcgc acctgattgc ccgacattat 5520
   ccgaatctgg caattccggt tcgcttgctg tccataaaac cgcccagtct agctatcgcc 5580
   atgtaagccc actgcaagct acctgctttc tctttgcgct tgcgttttcc ggatcttctt 5640
   gagatccttt ttttctgcgc gtaatctgct gcttgcaaac aaaaaaacca ccgctaccag 5700
   cggtggtttg tttgccggat caagagctac caactctttt tccgaaggta actggcttca 5760
   gcagagcgca gataccaaat actgtccttc tagtgtagcc gtagttaggc caccacttca 5820
   agaactctgt agcaccgcct acatacctcg ctctgctaat cctgttacca gtggctgctg 5880
   ccagtggcga taagtcgtgt cttaccgggt tggactcaag acgatagtta ccggataagg 5940
   cgcagcggtc gggctgaacg gggggttcgt gcacacagcc cagcttggag cgaacgacct 6000
   acaccgaact gagataccta cagcgtgagc tatgagaaag cgccacgctt cccgaaggga 6060
   gaaaggcgga caggtatccg gtaagcggca gggtcggaac aggagagcgc acgagggagc 6120
   ttccaggggg aaacgcctgg tatctttata gtcctgtcgg gtttcgccac ctctgacttg 6180
   agcgtcgatt tttgtgatgc tcgtcagggg ggcggagcct atggaaaaac gccagcaacg 6240
   cggccttttt acggttcctg gccttttgct ggccttttgc tcacatgttc tttcctgcgt 6300
   tatcccctga ttctgtggat aaccgtatta ccgcctttga gtgagctgat accgctcgcc 6360
   gcagccgaac gaccgagcgc agcgagtcag tgagcgagga agcggaagag cgcctgatgc 6420
   ggtattttct ccttacgcat ctgtgcggta tttcacaccg catatggtgc actctcagta 6480
   caatctgctc tgatgccgca tagttaagcc agtatacact ccgctatcgc tacgtgactg 6540
   ggtcatggct gcgccccgac acccgccaac acccgctgac gcgccctgac gggcttgtct 6600
   gctcccggca tccgcttaca gacaagctgt gaccgtctcc gggagctgca tgtgtcagag 6660
   gttttcaccg tcatcaccga aacgcgcgag gcagggtgcc ttgatgtggg cgccggcggt 6720
   cgagtggcga cggcgcggct tgtccgcgcc ctggtagatt gcctggccgt aggccagcca 6780
   tttttgagcg gccagcggcc gcgataggcc gacgcgaagc ggcggggcgt agggagcgca 6840
   gcgaccgaag ggtaggcgct tttgcagctc cttcggctgt gcgctggcca gacagttatg 6900
   cacaggccag gcgggtttta agagttttaa taagttttaa agagttttag gcggaaaaat 6960
   cgcctttttt ctcttttata tcagtcactt acatgtgtga ccggttccca atgtacggct 7020
   ttgggttccc aatgtacggg ttccggttcc caatgtacgg ctttgggttc ccaatgtacg 7080
   tgctatccac aggaaagaga ccttttcgac ctttttcccc tgctagggca atttgcccta 7140
   gcatctgctc cgtacattag gaaccggcgg atgcttcgcc ctcgatcagg ttgcggtagc 7200
   gcatgactag gatcgggcca gcctgccccg cctcctcctt caaatcgtac tccggcaggt 7260
   catttgaccc gatcagcttg cgcacggtga aacagaactt cttgaactct ccggcgctgc 7320
   cactgcgttc gtagatcgtc ttgaacaacc atctggcttc tgccttgcct gcggcgcggc 7380
   gtgccaggcg gtagagaaaa cggccgatgc cgggatcgat caaaaagtaa tcggggtgaa 7440
   ccgtcagcac gtccgggttc ttgccttctg tgatctcgcg gtacatccaa tcagctagct 7500
   cgatctcgat gtactccggc cgcccggttt cgctctttac gatcttgtag cggctaatca 7560
   aggcttcacc ctcggatacc gtcaccaggc ggccgttctt ggccttcttc gtacgctgca 7620
   tggcaacgtg cgtggtgttt aaccgaatgc aggtttctac caggtcgtct ttctgctttc 7680
   cgccatcggc tcgccggcag aacttgagta cgtccgcaac gtgtggacgg aacacgcggc 7740
   cgggcttgtc tcccttccct tcccggtatc ggttcatgga ttcggttaga tgggaaaccg 7800
   ccatcagtac caggtcgtaa tcccacacac tggccatgcc ggccggccct gcggaaacct 7860
   ctacgtgccc gtctggaagc tcgtagcgga tcacctcgcc agctcgtcgg tcacgcttcg 7920
   acagacggaa aacggccacg tccatgatgc tgcgactatc gcgggtgccc acgtcataga 7980
   gcatcggaac gaaaaaatct ggttgctcgt cgcccttggg cggcttccta atcgacggcg 8040
   caccggctgc cggcggttgc cgggattctt tgcggattcg atcagcggcc gcttgccacg 8100
   attcaccggg gcgtgcttct gcctcgatgc gttgccgctg ggcggcctgc gcggccttca 8160
   acttctccac caggtcatca cccagcgccg cgccgatttg taccgggccg gatggtttgc 8220
   gaccgtcacg ccgattcctc gggcttgggg gttccagtgc cattgcaggg ccggcagaca 8280
   acccagccgc ttacgcctgg ccaaccgccc gttcctccac acatggggca ttccacggcg 8340
   tcggtgcctg gttgttcttg attttccatg ccgcctcctt tagccgctaa aattcatcta 8400
   ctcatttatt catttgctca tttactctgg tagctgcgcg atgtattcag atagcagctc 8460
   ggtaatggtc ttgccttggc gtaccgcgta catcttcagc ttggtgtgat cctccgccgg 8520
   caactgaaag ttgacccgct tcatggctgg cgtgtctgcc aggctggcca acgttgcagc 8580
   cttgctgctg cgtgcgctcg gacggccggc acttagcgtg tttgtgcttt tgctcatttt 8640
   ctctttacct cattaactca aatgagtttt gatttaattt cagcggccag cgcctggacc 8700
   tcgcgggcag cgtcgccctc gggttctgat tcaagaacgg ttgtgccggc ggcggcagtg 8760
   cctgggtagc tcacgcgctg cgtgatacgg gactcaagaa tgggcagctc gtacccggcc 8820
   agcgcctcgg caacctcacc gccgatgcgc gtgcctttga tcgcccgcga cacgacaaag 8880
   gccgcttgta gccttccatc cgtgacctca atgcgctgct taaccagctc caccaggtcg 8940
   gcggtggccc atatgtcgta agggcttggc tgcaccggaa tcagcacgaa gtcggctgcc 9000
   ttgatcgcgg acacagccaa gtccgccgcc tggggcgctc cgtcgatcac tacgaagtcg 9060
   cgccggccga tggccttcac gtcgcggtca atcgtcgggc ggtcgatgcc gacaacggtt 9120
   agcggttgat cttcccgcac ggccgcccaa tcgcgggcac tgccctgggg atcggaatcg 9180
   actaacagaa catcggcccc ggcgagttgc agggcgcggg ctagatgggt tgcgatggtc 9240
   gtcttgcctg acccgccttt ctggttaagt acagcgataa ccttcatgcg ttccccttgc 9300
   gtatttgttt atttactcat cgcatcatat acgcagcgac cgcatgacgc aagctgtttt 9360
   actcaaatac acatcacctt tttagacggc ggcgctcggt ttcttcagcg gccaagctgg 9420
   ccggccaggc cgccagcttg gcatcagaca aaccggccag gatttcatgc agccgcacgg 9480
   ttgagacgtg cgcgggcggc tcgaacacgt acccggccgc gatcatctcc gcctcgatct 9540
   cttcggtaat gaaaaacggt tcgtcctggc cgtcctggtg cggtttcatg cttgttcctc 9600
   ttggcgttca ttctcggcgg ccgccagggc gtcggcctcg gtcaatgcgt cctcacggaa 9660
   ggcaccgcgc cgcctggcct cggtgggcgt cacttcctcg ctgcgctcaa gtgcgcggta 9720
   cagggtcgag cgatgcacgc caagcagtgc agccgcctct ttcacggtgc ggccttcctg 9780
   gtcgatcagc tcgcgggcgt gcgcgatctg tgccggggtg agggtagggc gggggccaaa 9840
   cttcacgcct cgggccttgg cggcctcgcg cccgctccgg gtgcggtcga tgattaggga 9900
   acgctcgaac tcggcaatgc cggcgaacac ggtcaacacc atgcggccgg ccggcgtggt 9960
   ggtgtcggcc cacggctctg ccaggctacg caggcccgcg ccggcctcct ggatgcgctc 10020
   ggcaatgtcc agtaggtcgc gggtgctgcg ggccaggcgg tctagcctgg tcactgtcac 10080
   aacgtcgcca gggcgtaggt ggtcaagcat cctggccagc tccgggcggt cgcgcctggt 10140
   gccggtgatc ttctcggaaa acagcttggt gcagccggcc gcgtgcagtt cggcccgttg 10200
   gttggtcaag tcctggtcgt cggtgctgac gcgggcatag cccagcaggc cagcggcggc 10260
   gctcttgttc atggcgtaat gtctccggtt ctagtcgcaa gtattctact ttatgcgact 10320
   aaaacacgcg acaagaaaac gccaggaaaa gggcagggcg gcagcctgtc gcgtaactta 10380
   ggacttgtgc gacatgtcgt tttcagaaga cggctgcact gaacgtcaga agccgactgc 10440
   actatagcag cggaggggtt ggatcgatcc ctgctcgcgc aggctgggtg ccaagctctc 10500
   gggtaacatc aaggcccgat ccttggagcc cttgccctcc cgcacgatga tcgtgccgtg 10560
   atcgaaatcc agatccttga cccgcagttg caaaccctca ctgatccgca tgcccgttcc 10620
   atacagaagc tgggcgaaca aacgatgctc gccttccaga aaaccgagga tgcgaaccac 10680
   ttcatccggg gtcagcacca ccggcaagcg cccggacggc cgaggtcttc cgatctcctg 10740
   aagccagggc agatccgtgc acagcacttg ccgtagaaga acagcaaggc cgccaatgcc 10800
   tgacgatgcg tggagaccga aaccttgcgc tcgttcgcca gccaggacag aaatgcctcg 10860
   acttcgctgc tgcccaaggt tgccgggtga cgcacaccgt ggaaacggat gaaggcacga 10920
   acccagtgga cataagcctg ttcggttcgt aagctgtaat gcaagtagcg tatgcgctca 10980
   cgcaactggt ccagaacctt gaccgaacgc agcggtggta acggcgcagt ggcggttttc 11040
   atggcttgtt atgactgttt ttttggggta cagtctatgc ctcgggcatc caagcagcaa 11100
   gcgcgttacg ccgtgggtcg atgtttgatg ttatggagca gcaacgatgt tacgcagcag 11160
   ggcagtcgcc ctaaaacaaa gttaaacatc atgagggaag cggtgatcgc cgaagtatcg 11220
   actcaactat cagaggtagt tggcgtcatc gagcgccatc tcgaaccgac gttgctggcc 11280
   gtacatttgt acggctccgc agtggatggc ggcctgaagc cacacagtga tattgatttg 11340
   ctggttacgg tgaccgtaag gcttgatgaa acaacgcggc gagctttgat caacgacctt 11400
   ttggaaactt cggcttcccc tggagagagc gagattctcc gcgctgtaga agtcaccatt 11460
   gttgtgcacg acgacatcat tccgtggcgt tatccagcta agcgcgaact gcaatttgga 11520
   gaatggcagc gcaatgacat tcttgcaggt atcttcgagc cagccacgat cgacattgat 11580
   ctggctatct tgctgacaaa agcaagagaa catagcgttg ccttggtagg tccagcggcg 11640
   gaggaactct ttgatccggt tcctgaacag gatctatttg aggcgctaaa tgaaacctta 11700
   acgctatgga actcgccgcc cgactgggct ggcgatgagc gaaatgtagt gcttacgttg 11760
   tcccgcattt ggtacagcgc agtaaccggc aaaatcgcgc cgaaggatgt cgctgccgac 11820
   tgggcaatgg agcgcctgcc ggcccagtat cagcccgtca tacttgaagc tagacaggct 11880
   tatcttggac aagaagaaga tcgcttggcc tcgcgcgcag atcagttgga agaatttgtc 11940
   cactacgtga aaggcgagat caccaaggta gtcggcaaat aatgtctaac aattcgttca 12000
   agccgacgcc gcttcgcggc gcggcttaac tcaagcgtta gatgcactaa gcacataatt 12060
   gctcacagcc aaactatcag gtcaagtctg cttttattat ttttaagcgt gcataataag 12120
   ccctacacaa attgggagat atatcatgaa aggctggctt tttcttgtta tcgcaatagt 12180
   tggcgaagta atcgcaacat ccgcattaaa atctagcgag ggctttacta agctagcttg 12240
   cttggtcgtt ccggtaccgt gaacgtcggc tcgattgtac ctgcgttcaa atactttgcg 12300
   atcgtgttgc gcgcctgccc ggtgcgtcgg ctgatctcac ggatcgactg cttctctcgc 12360
   aacgccatcc gacggatgat gtttaaaagt cccatgtgga tcactccgtt gccccgtcgc 12420
   tcaccgtgtt ggggggaagg tgcacatggc tcagttctca atggaaatta tctgcctaac 12480
   cggctcagtt ctgcgtagaa accaacatgc aagctccacc gggtgcaaag cggcagcggc 12540
   ggcaggatat attcaattgt aaatggctcc atggcgatcg ctacgtatct agaattcctg 12600
   caggtcgagt cgcgacgtac gttcgaacaa ttggttttaa a 12641
<210> 2
   <211> 12188
   <212> DNA
   <213> Artificial
<220>
   <223> vector
<400> 2
   cggcaggata tattcaattg taaatggctc catggcgatc gctacgcacg ctcgggtcgt      60
   tgggcagccc gatgacagcg accacgctct tgaagccctg tgcctccagg gacttcagca     120
   ggtgggtgta gagcgtggag cccagtcccg tccgctggtg gcggggggag acgtacacgg     180
   tcgactcggc cgtccagtcg taggcgttgc gtgccttcca ggggcccgcg taggcgatgc     240
   cggcgacctc gccgtccacc tcggcgacga gccagggata gcgctcccgc agacggacga     300
   ggtcgtccgt ccactcctgc ggttcctgcg gctcggtacg gaagttgacc gtgcttgtct     360
   cgatgtagtg gttgacgatg gtgcagaccg ccggcatgtc cgcctcggtg gcacggcgga     420
   tgtcggccgg gcgtcgttct gggtccatgg ttttggttta ataagaagag aaaagagttc     480
   ttttgttatg gctgaagtaa tagagaaatg agctcgagtc ctctccaaat gaaatgaact     540
   tccttatata gaggaagggt cttgcgaagg atagtgggat tgtgcgtcat cccttacgtc     600
   agtggagata tcacatcaat ccacttgctt tgaagacgtg gttggaacgt cttctttttc     660
   cacgatgctc ctcgtgggtg ggggtccatc tttgggacca ctgtcggcag aggcatcttg     720
   aacgatagcc tttcctttat cgcaatgatg gcatttgtag gtgccacctt ccttttctac     780
   tgtccttttg atgaagtgac agatagctgg gcaatggaat ccgaggaggt ttcccgatat     840
   taccctttgt tgaaaagtct caatagccct ttggtcttct gagactgtat ctttgatatt     900
   cttggagtag acgagagtgt cgtgctccac catgttgacg aagatttctt tcttgtcatt     960
   gagtcgtaaa agactctgta tgaactgttc gccagtcttc acggcgagtt ctgttagatc    1020
   ctcgatctga atttttgact ccatgtatgg tgcatatggc gcgcctaagc tagctagatc    1080
   atcaatttat gtattacaca taatatcgca ctcagtcttt catctacggc aatgtaccag    1140
   ctgatataat cagttattga aatatttctg aatttaaact tgcatcaata aatttatgtt    1200
   tttgcttgga ctataatacc tgacttgtta ttttatcaat aaatatttaa actatatttc    1260
   tttcaagata cgtatctaga attcgaaggt aattatccaa gatgtagcat caagaatcca    1320
   atgtttacgg gaaaaactat ggaagtatta tgtgagctca gcaagaagca gatcaatatg    1380
   cggcacatat gcaacctatg ttcaaaaatg aagaatgtac agatacaaga tcctatactg    1440
   ccagaatacg aagaagaata cgtagaaatt gaaaaagaag aaccaggcga agaaaagaat    1500
   cttgaagacg taagcactga cgacaacaat gaaaagaaga agataaggtc ggtgattgtg 1560
   aaagagacat agaggacaca tgtaaggtgg aaaatgtaag ggcggaaagt aaccttatca 1620
   caaaggaatc ttatccccca ctacttatcc ttttatattt ttccgtgtca tttttgccct 1680
   tgagttttcc tatataagga accaagttcg gcatttgtga aaacaagaaa aaatttggtg 1740
   taagctattt tctttgaagt actgaggata caacttcaga gaaatttgta agtttgtctc 1800
   gagatgaaaa agcctgaact caccgcgacg tctgtcgaga agtttctgat cgaaaagttc 1860
   gacagcgtct ccgacctgat gcagctctcg gagggcgaag aatctcgtgc tttcagcttc 1920
   gatgtaggag ggcgtggata tgtcctgcgg gtaaatagct gcgccgatgg tttctacaaa 1980
   gatcgttatg tttatcggca ctttgcatcg gccgcgctcc cgattccgga agtgcttgac 2040
   attggggagt tcagcgagag cctgacctat tgcatctccc gccgtgcaca gggtgtcacg 2100
   ttgcaagacc tgcctgaaac cgaactgccc gctgttctgc agccggtcgc ggaggccatg 2160
   gatgctatcg ctgcggccga tcttagccag acgagcgggt tcggcccatt cggaccgcaa 2220
   ggaatcggtc aatacactac atggcgtgat ttcatatgcg cgattgctga tccccatgtg 2280
   tatcactggc aaactgtgat ggacgacacc gtcagtgcgt ccgtcgcgca ggctctcgat 2340
   gagctgatgc tttgggccga ggactgcccc gaagtccggc acctcgtgca cgcggatttc 2400
   ggctccaaca atgtcctgac ggacaatggc cgcataacag cggtcattga ctggagcgag 2460
   gcgatgttcg gggattccca atacgaggtc gccaacatct tcttctggag gccgtggttg 2520
   gcttgtatgg agcagcagac gcgctacttc gagcggaggc atccggagct tgcaggatcg 2580
   ccgcgcctcc gggcgtatat gctccgcatt ggtcttgacc aactctatca gagcttggtt 2640
   gacggcaatt tcgatgatgc agcttgggcg cagggtcgat gcgacgcaat cgtccgatcc 2700
   ggagccggga ctgtcgggcg tacacaaatc gcccgcagaa gcgcggccgt ctggaccgat 2760
   ggctgtgtag aagtactcgc cgatagtgga aaccgacgcc ccagcactcg tccgagggca 2820
   aaggaatagg atatcaagct tggacacgct gaaatcacca gtctctctct acaaatctat 2880
   ctctctctat tttctccata ataatgtgtg agtagttccc agataaggga attagggttc 2940
   ctatagggtt tcgctcatgt gttgagcata taagaaaccc ttagtatgta tttgtatttg 3000
   taaaatactt ctatcaataa aatttctaat tcctaaaacc aaaatccagt actaaaatcc 3060
   agatctaact ataacggtcc taaggtagcg accgcgggag ccatttggga ggaaacacct 3120
   caaggcgatt gatgtcaaca tgaacaagat gcctgatgtc gccatgactc ttgctgtggt 3180
   tgccctcttt gccgatggcc cgacagccat cagagacgtg gcttcctgga gagtaaagga 3240
   gaccgagagg atggttgcga tccggacgga gctaaccaag ctgggagcat ctgttgagga 3300
   agggccggac tactgcatca tcacgccgcc ggagaagctg aacgtgacgg cgatcgacac 3360
   gtacgacgac cacaggatgg cgatggcttt ctcccttgcc gcctgtgccg aggtccccgt 3420
   caccatccgg gaccctgggt gcacccggaa gaccttcccc gactacttcg atgtgctgag 3480
   cactttcgtc aagaattaag ctctagaact agtggatccc ccgatccgcg tttgtgtttt 3540
   ctgggtttct cacttaagcg tctgcgtttt acttttgtat tgggtttggc gtttagtagt 3600
   ttgcggtagc gttcttgtta tgtgtaatta cgctttttct tcttgcttca gcagtttcgg 3660
   ttgaaatata aatcgaatca agtttcactt tatcagcgtt gttttaaatt ttggcattaa 3720
   attggtgaaa attgcttcaa ttttgtatct aaatagaaga gacaacatga aattcgactt 3780
   ttgacctcaa atcttcgaac atttatttcc tgatttcacg atggatgagg ataacgaaag 3840
   ggcggttcct atgtccggga aagttcccgt agaagacaat gagcaaagct actgaaacgc 3900
   ggacacgacg tcgcattggt acggatatga gttaaaccga ctcaattcct ttattaagac 3960
   ataaaccgat tttggttaaa gtgtaacagt gagctgatat aaaaccgaaa caaaccggta 4020
   caagtttgat tgagcaactt gatgacaaac ttcagaattt tggttattga atgaaaatca 4080
   tagtctaatc gtaaaaaatg tacagaagaa aagctagagc agaacaaaga ttctatattc 4140
   tggttccaat ttatcatcgc tttaacgtcc ctcagatttg atcgggctgc aggaattaaa 4200
   cgcccgggca cgtgggatcc tctagagtcg acggccgagt actggcagga tatataccgt 4260
   tgtaatttgt cgcgtgtgaa taagtcgctg tgtatgtttg tttgattgtt tctgttggag 4320
   tgcagcccat ttcaccggac aagtcggcta gattgattta gccctgatga actgccgagg 4380
   ggaagccatc ttgagcgcgg aatgggaatg gatttcgttg tacaacgaga cgacagaaca 4440
   cccacgggac cgagcttcga tcgagcatca aatgaaactg caatttattc atatcaggat 4500
   tatcaatacc atatttttga aaaagccgtt tctgtaatga aggagaaaac tcaccgaggc 4560
   agttccatag gatggcaaga tcctggtatc ggtctgcgat tccgactcgt ccaacatcaa 4620
   tacaacctat taatttcccc tcgtcaaaaa taaggttatc aagtgagaaa tcaccatgag 4680
   tgacgactga atccggtgag aatggcaaaa gtttatgcat ttctttccag acttgttcaa 4740
   caggccagcc attacgctcg tcatcaaaat cactcgcatc aaccaaaccg ttattcattc 4800
   gtgattgcgc ctgagcgaga cgaaatacgc cgctgttaaa aggacaatta caaacaggaa 4860
   tcgaatgcaa ccggcgcagg aacactgcca gcgcatcaac aatattttca cctgaatcag 4920
   gatattcttc taatacctgg aatgctgttt ttccggggat cgcagtggtg agtaaccatg 4980
   catcatcagg agtacggata aaatgcttga tggtcggaag aggcataaat tccgtcagcc 5040
   agtttagtct gaccatctca tctgtaacat cattggcaac gctacctttg ccatgtttca 5100
   gaaacaactc tggcgcatcg ggcttcccat acaatcgata gattgtcgca cctgattgcc 5160
   cgacattatc cgaatctggc aattccggtt cgcttgctgt ccataaaacc gcccagtcta 5220
   gctatcgcca tgtaagccca ctgcaagcta cctgctttct ctttgcgctt gcgttttccg 5280
   gatcttcttg agatcctttt tttctgcgcg taatctgctg cttgcaaaca aaaaaaccac 5340
   cgctaccagc ggtggtttgt ttgccggatc aagagctacc aactcttttt ccgaaggtaa 5400
   ctggcttcag cagagcgcag ataccaaata ctgtccttct agtgtagccg tagttaggcc 5460
   accacttcaa gaactctgta gcaccgccta catacctcgc tctgctaatc ctgttaccag 5520
   tggctgctgc cagtggcgat aagtcgtgtc ttaccgggtt ggactcaaga cgatagttac 5580
   cggataaggc gcagcggtcg ggctgaacgg ggggttcgtg cacacagccc agcttggagc 5640
   gaacgaccta caccgaactg agatacctac agcgtgagct atgagaaagc gccacgcttc 5700
   ccgaagggag aaaggcggac aggtatccgg taagcggcag ggtcggaaca ggagagcgca 5760
   cgagggagct tccaggggga aacgcctggt atctttatag tcctgtcggg tttcgccacc 5820
   tctgacttga gcgtcgattt ttgtgatgct cgtcaggggg gcggagccta tggaaaaacg 5880
   ccagcaacgc ggccttttta cggttcctgg ccttttgctg gccttttgct cacatgttct 5940
   ttcctgcgtt atcccctgat tctgtggata accgtattac cgcctttgag tgagctgata 6000
   ccgctcgccg cagccgaacg accgagcgca gcgagtcagt gagcgaggaa gcggaagagc 6060
   gcctgatgcg gtattttctc cttacgcatc tgtgcggtat ttcacaccgc atatggtgca 6120
   ctctcagtac aatctgctct gatgccgcat agttaagcca gtatacactc cgctatcgct 6180
   acgtgactgg gtcatggctg cgccccgaca cccgccaaca cccgctgacg cgccctgacg 6240
   ggcttgtctg ctcccggcat ccgcttacag acaagctgtg accgtctccg ggagctgcat 6300
   gtgtcagagg ttttcaccgt catcaccgaa acgcgcgagg cagggtgcct tgatgtgggc 6360
   gccggcggtc gagtggcgac ggcgcggctt gtccgcgccc tggtagattg cctggccgta 6420
   ggccagccat ttttgagcgg ccagcggccg cgataggccg acgcgaagcg gcggggcgta 6480
   gggagcgcag cgaccgaagg gtaggcgctt tttgcagctc ttcggctgtg cgctggccag 6540
   acagttatgc acaggccagg cgggttttaa gagttttaat aagttttaaa gagttttagg 6600
   cggaaaaatc gccttttttc tcttttatat cagtcactta catgtgtgac cggttcccaa 6660
   tgtacggctt tgggttccca atgtacgggt tccggttccc aatgtacggc tttgggttcc 6720
   caatgtacgt gctatccaca ggaaagagac cttttcgacc tttttcccct gctagggcaa 6780
   tttgccctag catctgctcc gtacattagg aaccggcgga tgcttcgccc tcgatcaggt 6840
   tgcggtagcg catgactagg atcgggccag cctgccccgc ctcctccttc aaatcgtact 6900
   ccggcaggtc atttgacccg atcagcttgc gcacggtgaa acagaacttc ttgaactctc 6960
   cggcgctgcc actgcgttcg tagatcgtct tgaacaacca tctggcttct gccttgcctg 7020
   cggcgcggcg tgccaggcgg tagagaaaac ggccgatgcc gggatcgatc aaaaagtaat 7080
   cggggtgaac cgtcagcacg tccgggttct tgccttctgt gatctcgcgg tacatccaat 7140
   cagctagctc gatctcgatg tactccggcc gcccggtttc gctctttacg atcttgtagc 7200
   ggctaatcaa ggcttcaccc tcggataccg tcaccaggcg gccgttcttg gccttcttcg 7260
   tacgctgcat ggcaacgtgc gtggtgttta accgaatgca ggtttctacc aggtcgtctt 7320
   tctgctttcc gccatcggct cgccggcaga acttgagtac gtccgcaacg tgtggacgga 7380
   acacgcggcc gggcttgtct cccttccctt cccggtatcg gttcatggat tcggttagat 7440
   gggaaaccgc catcagtacc aggtcgtaat cccacacact ggccatgccg gccggccctg 7500
   cggaaacctc tacgtgcccg tctggaagct cgtagcggat cacctcgcca gctcgtcggt 7560
   cacgcttcga cagacggaaa acggccacgt ccatgatgct gcgactatcg cgggtgccca 7620
   cgtcatagag catcggaacg aaaaaatctg gttgctcgtc gcccttgggc ggcttcctaa 7680
   tcgacggcgc accggctgcc ggcggttgcc gggattcttt gcggattcga tcagcggccg 7740
   cttgccacga ttcaccgggg cgtgcttctg cctcgatgcg ttgccgctgg gcggcctgcg 7800
   cggccttcaa cttctccacc aggtcatcac ccagcgccgc gccgatttgt accgggccgg 7860
   atggtttgcg accgtcacgc cgattcctcg ggcttggggg ttccagtgcc attgcagggc 7920
   cggcagacaa cccagccgct tacgcctggc caaccgcccg ttcctccaca catggggcat 7980
   tccacggcgt cggtgcctgg ttgttcttga ttttccatgc cgcctccttt agccgctaaa 8040
   attcatctac tcatttattc atttgctcat ttactctggt agctgcgcga tgtattcaga 8100
   tagcagctcg gtaatggtct tgccttggcg taccgcgtac atcttcagct tggtgtgatc 8160
   ctccgccggc aactgaaagt tgacccgctt catggctggc gtgtctgcca ggctggccaa 8220
   cgttgcagcc ttgctgctgc gtgcgctcgg acggccggca cttagcgtgt ttgtgctttt 8280
   gctcattttc tctttacctc attaactcaa atgagttttg atttaatttc agcggccagc 8340
   gcctggacct cgcgggcagc gtcgccctcg ggttctgatt caagaacggt tgtgccggcg 8400
   gcggcagtgc ctgggtagct cacgcgctgc gtgatacggg actcaagaat gggcagctcg 8460
   tacccggcca gcgcctcggc aacctcaccg ccgatgcgcg tgcctttgat cgcccgcgac 8520
   acgacaaagg ccgcttgtag ccttccatcc gtgacctcaa tgcgctgctt aaccagctcc 8580
   accaggtcgg cggtggccca tatgtcgtaa gggcttggct gcaccggaat cagcacgaag 8640
   tcggctgcct tgatcgcgga cacagccaag tccgccgcct ggggcgctcc gtcgatcact 8700
   acgaagtcgc gccggccgat ggccttcacg tcgcggtcaa tcgtcgggcg gtcgatgccg 8760
   acaacggtta gcggttgatc ttcccgcacg gccgcccaat cgcgggcact gccctgggga 8820
   tcggaatcga ctaacagaac atcggccccg gcgagttgca gggcgcgggc tagatgggtt 8880
   gcgatggtcg tcttgcctga cccgcctttc tggttaagta cagcgataac cttcatgcgt 8940
   tccccttgcg tatttgttta tttactcatc gcatcatata cgcagcgacc gcatgacgca 9000
   agctgtttta ctcaaataca catcaccttt ttagacggcg gcgctcggtt tcttcagcgg 9060
   ccaagctggc cggccaggcc gccagcttgg catcagacaa accggccagg atttcatgca 9120
   gccgcacggt tgagacgtgc gcgggcggct cgaacacgta cccggccgcg atcatctccg 9180
   cctcgatctc ttcggtaatg aaaaacggtt cgtcctggcc gtcctggtgc ggtttcatgc 9240
   ttgttcctct tggcgttcat tctcggcggc cgccagggcg tcggcctcgg tcaatgcgtc 9300
   ctcacggaag gcaccgcgcc gcctggcctc ggtgggcgtc acttcctcgc tgcgctcaag 9360
   tgcgcggtac agggtcgagc gatgcacgcc aagcagtgca gccgcctctt tcacggtgcg 9420
   gccttcctgg tcgatcagct cgcgggcgtg cgcgatctgt gccggggtga gggtagggcg 9480
   ggggccaaac ttcacgcctc gggccttggc ggcctcgcgc ccgctccggg tgcggtcgat 9540
   gattagggaa cgctcgaact cggcaatgcc ggcgaacacg gtcaacacca tgcggccggc 9600
   cggcgtggtg gtgtcggccc acggctctgc caggctacgc aggcccgcgc cggcctcctg 9660
   gatgcgctcg gcaatgtcca gtaggtcgcg ggtgctgcgg gccaggcggt ctagcctggt 9720
   cactgtcaca acgtcgccag ggcgtaggtg gtcaagcatc ctggccagct ccgggcggtc 9780
   gcgcctggtg ccggtgatct tctcggaaaa cagcttggtg cagccggccg cgtgcagttc 9840
   ggcccgttgg ttggtcaagt cctggtcgtc ggtgctgacg cgggcatagc ccagcaggcc 9900
   agcggcggcg ctcttgttca tggcgtaatg tctccggttc tagtcgcaag tattctactt 9960
   tatgcgacta aaacacgcga caagaaaacg ccaggaaaag ggcagggcgg cagcctgtcg 10020
   cgtaacttag gacttgtgcg acatgtcgtt ttcagaagac ggctgcactg aacgtcagaa 10080
   gccgactgca ctatagcagc ggaggggttg gatcgatccc tgctcgcgca ggctgggtgc 10140
   caagctctcg ggtaacatca aggcccgatc cttggagccc ttgccctccc gcacgatgat 10200
   cgtgccgtga tcgaaatcca gatccttgac ccgcagttgc aaaccctcac tgatccgcat 10260
   gcccgttcca tacagaagct gggcgaacaa acgatgctcg ccttccagaa aaccgaggat 10320
   gcgaaccact tcatccgggg tcagcaccac cggcaagcgc ccggacggcc gaggtcttcc 10380
   gatctcctga agccagggca gatccgtgca cagcacttgc cgtagaagaa cagcaaggcc 10440
   gccaatgcct gacgatgcgt ggagaccgaa accttgcgct cgttcgccag ccaggacaga 10500
   aatgcctcga cttcgctgct gcccaaggtt gccgggtgac gcacaccgtg gaaacggatg 10560
   aaggcacgaa cccagtggac ataagcctgt tcggttcgta agctgtaatg caagtagcgt 10620
   atgcgctcac gcaactggtc cagaaccttg accgaacgca gcggtggtaa cggcgcagtg 10680
   gcggttttca tggcttgtta tgactgtttt tttggggtac agtctatgcc tcgggcatcc 10740
   aagcagcaag cgcgttacgc cgtgggtcga tgtttgatgt tatggagcag caacgatgtt 10800
   acgcagcagg gcagtcgccc taaaacaaag ttaaacatca tgagggaagc ggtgatcgcc 10860
   gaagtatcga ctcaactatc agaggtagtt ggcgtcatcg agcgccatct cgaaccgacg 10920
   ttgctggccg tacatttgta cggctccgca gtggatggcg gcctgaagcc acacagtgat 10980
   attgatttgc tggttacggt gaccgtaagg cttgatgaaa caacgcggcg agctttgatc  11040
   aacgaccttt tggaaacttc ggcttcccct ggagagagcg agattctccg cgctgtagaa  11100
   gtcaccattg ttgtgcacga cgacatcatt ccgtggcgtt atccagctaa gcgcgaactg  11160
   caatttggag aatggcagcg caatgacatt cttgcaggta tcttcgagcc agccacgatc  11220
   gacattgatc tggctatctt gctgacaaaa gcaagagaac atagcgttgc cttggtaggt  11280
   ccagcggcgg aggaactctt tgatccggtt cctgaacagg atctatttga ggcgctaaat  11340
   gaaaccttaa cgctatggaa ctcgccgccc gactgggctg gcgatgagcg aaatgtagtg  11400
   cttacgttgt cccgcatttg gtacagcgca gtaaccggca aaatcgcgcc gaaggatgtc  11460
   gctgccgact gggcaatgga gcgcctgccg gcccagtatc agcccgtcat acttgaagct  11520
   agacaggctt atcttggaca agaagaagat cgcttggcct cgcgcgcaga tcagttggaa  11580
   gaatttgtcc actacgtgaa aggcgagatc accaaggtag tcggcaaata atgtctaaca  11640
   attcgttcaa gccgacgccg cttcgcggcg cggcttaact caagcgttag atgcactaag  11700
   cacataattg ctcacagcca aactatcagg tcaagtctgc ttttattatt tttaagcgtg  11760
   cataataagc cctacacaaa ttgggagata tatcatgaaa ggctggcttt ttcttgttat  11820
   cgcaatagtt ggcgaagtaa tcgcaacatc cgcattaaaa tctagcgagg gctttactaa  11880
   gctagcttgc ttggtcgttc cggtaccgtg aacgtcggct cgattgtacc tgcgttcaaa  11940
   tactttgcga tcgtgttgcg cgcctgcccg gtgcgtcggc tgatctcacg gatcgactgc  12000
   ttctctcgca acgccatccg acggatgatg tttaaaagtc ccatgtggat cactccgttg  12060
   ccccgtcgct caccgtgttg gggggaaggt gcacatggct cagttctcaa tggaaattat  12120
   ctgcctaacc ggctcagttc tgcgtagaaa ccaacatgca agctccaccg ggtgcaaagc  12180
   ggcagcgg                                                            12188
<210> 3
   <211> 11500
   <212> DNA
   <213> Artificial
<220>
   <223> vector
<400> 3
   cggcaggata tattcaattg taaatggctc catggcgatc gctaccctct gcagtcgacg     60
   ggcccgttgt cccgcggtcg ctaccttagg accgttatag ttagatctgg attttagtac    120
   tggattttgg ttttaggaat tagaaatttt attgatagaa gtattttaca aatacaaata    180
   catactaagg gtttcttata tgctcaacac atgagcgaaa ccctatagga accctaattc    240
   ccttatctgg gaactactca cacattatta tggagaaaat agagagagat agatttgtag    300
   agagagactg gtgatttcag cgtgtccaag cttgatatcc tattcctttg ccctcggacg 360
   agtgctgggg cgtcggtttc cactatcggc gagtacttct acacagccat cggtccagac 420
   ggccgcgctt ctgcgggcga tttgtgtacg cccgacagtc ccggctccgg atcggacgat 480
   tgcgtcgcat cgaccctgcg cccaagctgc atcatcgaaa ttgccgtcaa ccaagctctg 540
   atagagttgg tcaagaccaa tgcggagcat atacgcccgg aggcgcggcg atcctgcaag 600
   ctccggatgc ctccgctcga agtagcgcgt ctgctgctcc atacaagcca accacggcct 660
   ccagaagaag atgttggcga cctcgtattg ggaatccccg aacatcgcct cgctccagtc 720
   aatgaccgct gttatgcggc cattgtccgt caggacattg ttggagccga aatccgcgtg 780
   cacgaggtgc cggacttcgg ggcagtcctc ggcccaaagc atcagctcat cgagagcctg 840
   cgcgacggac gcactgacgg tgtcgtccat cacagtttgc cagtgataca catggggatc 900
   agcaatcgcg catatgaaat cacgccatgt agtgtattga ccgattcctt gcggtccgaa 960
   tgggccgaac ccgctcgtct ggctaagatc ggccgcagcg atagcatcca tggcctccgc 1020
   gaccggctgc agaacagcgg gcagttcggt ttcaggcagg tcttgcaacg tgacaccctg 1080
   tgcacggcgg gagatgcaat aggtcaggct ctcgctgaac tccccaatgt caagcacttc 1140
   cggaatcggg agcgcggccg atgcaaagtg ccgataaaca taacgatctt tgtagaaacc 1200
   atcggcgcag ctatttaccc gcaggacata tccacgccct cctacatcga agctgaaagc 1260
   acgagattct tcgccctccg agagctgcat caggtcggag acgctgtcga acttttcgat 1320
   cagaaacttc tcgacagacg tcgcggtgag ttcaggcttt ttcatctcga gacaaactta 1380
   caaatttctc tgaagttgta tcctcagtac ttcaaagaaa atagcttaca ccaaattttt 1440
   tcttgttttc acaaatgccg aacttggttc cttatatagg aaaactcaag ggcaaaaatg 1500
   acacggaaaa atataaaagg ataagtagtg ggggataaga ttcctttgtg ataaggttac 1560
   tttccgccct tacattttcc accttacatg tgtcctctat gtctctttca caatcaccga 1620
   ccttatcttc ttcttttcat tgttgtcgtc agtgcttacg tcttcaagat tcttttcttc 1680
   gcctggttct tctttttcaa tttctacgta ttcttcttcg tattctggca gtataggatc 1740
   ttgtatctgt acattcttca tttttgaaca taggttgcat atgtgccgca tattgatctg 1800
   cttcttgctg agctcacata atacttccat agtttttccc gtaaacattg gattcttgat 1860
   gctacatctt ggataattac cttcgaattc gtcttgggga tccgatatct agagtatacg 1920
   cgttaacgcg gccgctgtac catgcatgat ctggatttta gtactggatt ttggttttag 1980
   gaattagaaa ttttattgat agaagtattt tacaaataca aatacatact aagggtttct 2040
   tatatgctca acacatgagc gaaaccctat aggaacccta attcccttat ctgggaacta 2100
   ctcacacatt attatggaga aaatagagag agatagattt gtagagagag actggtgatt 2160
   tcagcgtgtc caagcttgct agccctattt caggaaagtt tcggaggaga tagtgttcgg 2220
   cagtttgtac atcatctgcg ggatcaggta cggtttgatc aggttgtaga agatcaggta 2280
   agacatagaa tcgatgtaga tgatcggttt gtttttgttg atttttacgt aacagttcag 2340
   ttggaatttg ttacgcagac ccttaaccag gtattctact tcttcgaaag tgaaagactg 2400
   ggtgttcagt acgatcgatt tgttggtaga gtttttgttg taatcccatt taccaccatc 2460
   atccatgaac cagtatgcca gagacatcgg ggtcaggtag ttttcaacca ggttgttcgg 2520
   gatggttttt ttgttgttaa cgatgaacag gttagccagt ttgttgaaag cttggtgttt 2580
   gaaagtctgg gcgccccagg tgattaccag gttacccagg tggttaacac gttctttttt 2640
   gtgcggcggg gacagtaccc actgatcgta cagcagacat acgtggtcca tgtatgcttt 2700
   gtttttccac tcgaactgca tacagtaggt tttaccttca tcacgagaac ggatgtaagc 2760
   atcacccagg atcagaccga tacctgcttc gaactgttcg atgttcagtt cgatcagctg 2820
   ggatttgtat tctttcagca gtttagagtt cggacccagg ttcattacct ggtttttttt 2880
   gatgttaacc ttgcgcttct tcttgggggg tttagccatg gttttggact tcttcttctt 2940
   cttcttttgc ttaattctcg agtcctctcc aaatgaaatg aacttcctta tatagaggaa 3000
   gggtcttgcg aaggatagtg ggattgtgcg tcatccctta cgtcagtgga gatatcacat 3060
   caatccactt gctttgaaga cgtggttgga acgtcttctt tttccacgat gctcctcgtg 3120
   ggtgggggtc catctttggg accactgtcg gcagaggcat cttgaacgat agcctttcct 3180
   ttatcgcaat gatggcattt gtaggtgcca ccttcctttt ctactgtcct tttgatgaag 3240
   tgacagatag ctgggcaatg gaatccgagg aggtttcccg atattaccct ttgttgaaaa 3300
   gtctcaatag ccctttggtc ttctgagact gtatctttga tattcttgga gtagacgaga 3360
   gtgtcgtgct ccaccatgtt gacgaagatt ttcttcttgt cattgagtcg taaaagactc 3420
   tgtatgaact gttcgccagt cttcacggcg agttctgtta gatcctcgat ctgaattttt 3480
   gactccatgt atggtgcata tggcgcgcca tgcatacgta ggtaccaatt gcctgcaggt 3540
   cgacggccga gtactggcag gatatatacc gttgtaattt gtcgcgtgtg aataagtcgc 3600
   tgtgtatgtt tgtttgattg tttctgttgg agtgcagccc atttcaccgg acaagtcggc 3660
   tagattgatt tagccctgat gaactgccga ggggaagcca tcttgagcgc ggaatgggaa 3720
   tggatttcgt tgtacaacga gacgacagaa cacccacggg accgagcttc gatcgagcat 3780
   caaatgaaac tgcaatttat tcatatcagg attatcaata ccatattttt gaaaaagccg 3840
   tttctgtaat gaaggagaaa actcaccgag gcagttccat aggatggcaa gatcctggta 3900
   tcggtctgcg attccgactc gtccaacatc aatacaacct attaatttcc cctcgtcaaa 3960
   aataaggtta tcaagtgaga aatcaccatg agtgacgact gaatccggtg agaatggcaa 4020
   aagtttatgc atttctttcc agacttgttc aacaggccag ccattacgct cgtcatcaaa 4080
   atcactcgca tcaaccaaac cgttattcat tcgtgattgc gcctgagcga gacgaaatac 4140
   gccgctgtta aaaggacaat tacaaacagg aatcgaatgc aaccggcgca ggaacactgc 4200
   cagcgcatca acaatatttt cacctgaatc aggatattct tctaatacct ggaatgctgt 4260
   ttttccgggg atcgcagtgg tgagtaacca tgcatcatca ggagtacgga taaaatgctt 4320
   gatggtcgga agaggcataa attccgtcag ccagtttagt ctgaccatct catctgtaac 4380
   atcattggca acgctacctt tgccatgttt cagaaacaac tctggcgcat cgggcttccc 4440
   atacaatcga tagattgtcg cacctgattg cccgacatta tccgaatctg gcaattccgg 4500
   ttcgcttgct gtccataaaa ccgcccagtc tagctatcgc catgtaagcc cactgcaagc 4560
   tacctgcttt ctctttgcgc ttgcgttttc cggatcttct tgagatcctt tttttctgcg 4620
   cgtaatctgc tgcttgcaaa caaaaaaacc accgctacca gcggtggttt gtttgccgga 4680
   tcaagagcta ccaactcttt ttccgaaggt aactggcttc agcagagcgc agataccaaa 4740
   tactgtcctt ctagtgtagc cgtagttagg ccaccacttc aagaactctg tagcaccgcc 4800
   tacatacctc gctctgctaa tcctgttacc agtggctgct gccagtggcg ataagtcgtg 4860
   tcttaccggg ttggactcaa gacgatagtt accggataag gcgcagcggt cgggctgaac 4920
   ggggggttcg tgcacacagc ccagcttgga gcgaacgacc tacaccgaac tgagatacct 4980
   acagcgtgag ctatgagaaa gcgccacgct tcccgaaggg agaaaggcgg acaggtatcc 5040
   ggtaagcggc agggtcggaa caggagagcg cacgagggag cttccagggg gaaacgcctg 5100
   gtatctttat agtcctgtcg ggtttcgcca cctctgactt gagcgtcgat ttttgtgatg 5160
   ctcgtcaggg gggcggagcc tatggaaaaa cgccagcaac gcggcctttt tacggttcct 5220
   ggccttttgc tggccttttg ctcacatgtt ctttcctgcg ttatcccctg attctgtgga 5280
   taaccgtatt accgcctttg agtgagctga taccgctcgc cgcagccgaa cgaccgagcg 5340
   cagcgagtca gtgagcgagg aagcggaaga gcgcctgatg cggtattttc tccttacgca 5400
   tctgtgcggt atttcacacc gcatatggtg cactctcagt acaatctgct ctgatgccgc 5460
   atagttaagc cagtatacac tccgctatcg ctacgtgact gggtcatggc tgcgccccga 5520
   cacccgccaa cacccgctga cgcgccctga cgggcttgtc tgctcccggc atccgcttac 5580
   agacaagctg tgaccgtctc cgggagctgc atgtgtcaga ggttttcacc gtcatcaccg 5640
   aaacgcgcga ggcagggtgc cttgatgtgg gcgccggcgg tcgagtggcg acggcgcggc 5700
   ttgtccgcgc cctggtagat tgcctggccg taggccagcc atttttgagc ggccagcggc 5760
   cgcgataggc cgacgcgaag cggcggggcg tagggagcgc agcgaccgaa gggtaggcgc 5820
   tttttgcagc tcttcggctg tgcgctggcc agacagttat gcacaggcca ggcgggtttt 5880
   aagagtttta ataagtttta aagagtttta ggcggaaaaa tcgccttttt tctcttttat 5940
   atcagtcact tacatgtgtg accggttccc aatgtacggc tttgggttcc caatgtacgg 6000
   gttccggttc ccaatgtacg gctttgggtt cccaatgtac gtgctatcca caggaaagag 6060
   accttttcga cctttttccc ctgctagggc aatttgccct agcatctgct ccgtacatta 6120
   ggaaccggcg gatgcttcgc cctcgatcag gttgcggtag cgcatgacta ggatcgggcc 6180
   agcctgcccc gcctcctcct tcaaatcgta ctccggcagg tcatttgacc cgatcagctt 6240
   gcgcacggtg aaacagaact tcttgaactc tccggcgctg ccactgcgtt cgtagatcgt 6300
   cttgaacaac catctggctt ctgccttgcc tgcggcgcgg cgtgccaggc ggtagagaaa 6360
   acggccgatg ccgggatcga tcaaaaagta atcggggtga accgtcagca cgtccgggtt 6420
   cttgccttct gtgatctcgc ggtacatcca atcagctagc tcgatctcga tgtactccgg 6480
   ccgcccggtt tcgctcttta cgatcttgta gcggctaatc aaggcttcac cctcggatac 6540
   cgtcaccagg cggccgttct tggccttctt cgtacgctgc atggcaacgt gcgtggtgtt 6600
   taaccgaatg caggtttcta ccaggtcgtc tttctgcttt ccgccatcgg ctcgccggca 6660
   gaacttgagt acgtccgcaa cgtgtggacg gaacacgcgg ccgggcttgt ctcccttccc 6720
   ttcccggtat cggttcatgg attcggttag atgggaaacc gccatcagta ccaggtcgta 6780
   atcccacaca ctggccatgc cggccggccc tgcggaaacc tctacgtgcc cgtctggaag 6840
   ctcgtagcgg atcacctcgc cagctcgtcg gtcacgcttc gacagacgga aaacggccac 6900
   gtccatgatg ctgcgactat cgcgggtgcc cacgtcatag agcatcggaa cgaaaaaatc 6960
   tggttgctcg tcgcccttgg gcggcttcct aatcgacggc gcaccggctg ccggcggttg 7020
   ccgggattct ttgcggattc gatcagcggc cgcttgccac gattcaccgg ggcgtgcttc 7080
   tgcctcgatg cgttgccgct gggcggcctg cgcggccttc aacttctcca ccaggtcatc 7140
   acccagcgcc gcgccgattt gtaccgggcc ggatggtttg cgaccgtcac gccgattcct 7200
   cgggcttggg ggttccagtg ccattgcagg gccggcagac aacccagccg cttacgcctg 7260
   gccaaccgcc cgttcctcca cacatggggc attccacggc gtcggtgcct ggttgttctt 7320
   gattttccat gccgcctcct ttagccgcta aaattcatct actcatttat tcatttgctc 7380
   atttactctg gtagctgcgc gatgtattca gatagcagct cggtaatggt cttgccttgg 7440
   cgtaccgcgt acatcttcag cttggtgtga tcctccgccg gcaactgaaa gttgacccgc 7500
   ttcatggctg gcgtgtctgc caggctggcc aacgttgcag ccttgctgct gcgtgcgctc 7560
   ggacggccgg cacttagcgt gtttgtgctt ttgctcattt tctctttacc tcattaactc 7620
   aaatgagttt tgatttaatt tcagcggcca gcgcctggac ctcgcgggca gcgtcgccct 7680
   cgggttctga ttcaagaacg gttgtgccgg cggcggcagt gcctgggtag ctcacgcgct 7740
   gcgtgatacg ggactcaaga atgggcagct cgtacccggc cagcgcctcg gcaacctcac 7800
   cgccgatgcg cgtgcctttg atcgcccgcg acacgacaaa ggccgcttgt agccttccat 7860
   ccgtgacctc aatgcgctgc ttaaccagct ccaccaggtc ggcggtggcc catatgtcgt 7920
   aagggcttgg ctgcaccgga atcagcacga agtcggctgc cttgatcgcg gacacagcca 7980
   agtccgccgc ctggggcgct ccgtcgatca ctacgaagtc gcgccggccg atggccttca 8040
   cgtcgcggtc aatcgtcggg cggtcgatgc cgacaacggt tagcggttga tcttcccgca 8100
   cggccgccca atcgcgggca ctgccctggg gatcggaatc gactaacaga acatcggccc 8160
   cggcgagttg cagggcgcgg gctagatggg ttgcgatggt cgtcttgcct gacccgcctt 8220
   tctggttaag tacagcgata accttcatgc gttccccttg cgtatttgtt tatttactca 8280
   tcgcatcata tacgcagcga ccgcatgacg caagctgttt tactcaaata cacatcacct 8340
   ttttagacgg cggcgctcgg tttcttcagc ggccaagctg gccggccagg ccgccagctt 8400
   ggcatcagac aaaccggcca ggatttcatg cagccgcacg gttgagacgt gcgcgggcgg 8460
   ctcgaacacg tacccggccg cgatcatctc cgcctcgatc tcttcggtaa tgaaaaacgg 8520
   ttcgtcctgg ccgtcctggt gcggtttcat gcttgttcct cttggcgttc attctcggcg 8580
   gccgccaggg cgtcggcctc ggtcaatgcg tcctcacgga aggcaccgcg ccgcctggcc 8640
   tcggtgggcg tcacttcctc gctgcgctca agtgcgcggt acagggtcga gcgatgcacg 8700
   ccaagcagtg cagccgcctc tttcacggtg cggccttcct ggtcgatcag ctcgcgggcg 8760
   tgcgcgatct gtgccggggt gagggtaggg cgggggccaa acttcacgcc tcgggccttg 8820
   gcggcctcgc gcccgctccg ggtgcggtcg atgattaggg aacgctcgaa ctcggcaatg 8880
   ccggcgaaca cggtcaacac catgcggccg gccggcgtgg tggtgtcggc ccacggctct 8940
   gccaggctac gcaggcccgc gccggcctcc tggatgcgct cggcaatgtc cagtaggtcg 9000
   cgggtgctgc gggccaggcg gtctagcctg gtcactgtca caacgtcgcc agggcgtagg 9060
   tggtcaagca tcctggccag ctccgggcgg tcgcgcctgg tgccggtgat cttctcggaa 9120
   aacagcttgg tgcagccggc cgcgtgcagt tcggcccgtt ggttggtcaa gtcctggtcg 9180
   tcggtgctga cgcgggcata gcccagcagg ccagcggcgg cgctcttgtt catggcgtaa 9240
   tgtctccggt tctagtcgca agtattctac tttatgcgac taaaacacgc gacaagaaaa 9300
   cgccaggaaa agggcagggc ggcagcctgt cgcgtaactt aggacttgtg cgacatgtcg 9360
   ttttcagaag acggctgcac tgaacgtcag aagccgactg cactatagca gcggaggggt 9420
   tggatcgatc cctgctcgcg caggctgggt gccaagctct cgggtaacat caaggcccga 9480
   tccttggagc ccttgccctc ccgcacgatg atcgtgccgt gatcgaaatc cagatccttg 9540
   acccgcagtt gcaaaccctc actgatccgc atgcccgttc catacagaag ctgggcgaac 9600
   aaacgatgct cgccttccag aaaaccgagg atgcgaacca cttcatccgg ggtcagcacc 9660
   accggcaagc gcccggacgg ccgaggtctt ccgatctcct gaagccaggg cagatccgtg 9720
   cacagcactt gccgtagaag aacagcaagg ccgccaatgc ctgacgatgc gtggagaccg 9780
   aaaccttgcg ctcgttcgcc agccaggaca gaaatgcctc gacttcgctg ctgcccaagg 9840
   ttgccgggtg acgcacaccg tggaaacgga tgaaggcacg aacccagtgg acataagcct 9900
   gttcggttcg taagctgtaa tgcaagtagc gtatgcgctc acgcaactgg tccagaacct 9960
   tgaccgaacg cagcggtggt aacggcgcag tggcggtttt catggcttgt tatgactgtt 10020
   tttttggggt acagtctatg cctcgggcat ccaagcagca agcgcgttac gccgtgggtc 10080
   gatgtttgat gttatggagc agcaacgatg ttacgcagca gggcagtcgc cctaaaacaa 10140
   agttaaacat catgagggaa gcggtgatcg ccgaagtatc gactcaacta tcagaggtag 10200
   ttggcgtcat cgagcgccat ctcgaaccga cgttgctggc cgtacatttg tacggctccg 10260
   cagtggatgg cggcctgaag ccacacagtg atattgattt gctggttacg gtgaccgtaa 10320
   ggcttgatga aacaacgcgg cgagctttga tcaacgacct tttggaaact tcggcttccc 10380
   ctggagagag cgagattctc cgcgctgtag aagtcaccat tgttgtgcac gacgacatca 10440
   ttccgtggcg ttatccagct aagcgcgaac tgcaatttgg agaatggcag cgcaatgaca 10500
   ttcttgcagg tatcttcgag ccagccacga tcgacattga tctggctatc ttgctgacaa 10560
   aagcaagaga acatagcgtt gccttggtag gtccagcggc ggaggaactc tttgatccgg 10620
   ttcctgaaca ggatctattt gaggcgctaa atgaaacctt aacgctatgg aactcgccgc 10680
   ccgactgggc tggcgatgag cgaaatgtag tgcttacgtt gtcccgcatt tggtacagcg 10740
   cagtaaccgg caaaatcgcg ccgaaggatg tcgctgccga ctgggcaatg gagcgcctgc 10800
   cggcccagta tcagcccgtc atacttgaag ctagacaggc ttatcttgga caagaagaag 10860
   atcgcttggc ctcgcgcgca gatcagttgg aagaatttgt ccactacgtg aaaggcgaga 10920
   tcaccaaggt agtcggcaaa taatgtctaa caattcgttc aagccgacgc cgcttcgcgg 10980
   cgcggcttaa ctcaagcgtt agatgcacta agcacataat tgctcacagc caaactatca 11040
   ggtcaagtct gcttttatta tttttaagcg tgcataataa gccctacaca aattgggaga 11100
   tatatcatga aaggctggct ttttcttgtt atcgcaatag ttggcgaagt aatcgcaaca 11160
   tccgcattaa aatctagcga gggctttact aagctagctt gcttggtcgt tccggtaccg 11220
   tgaacgtcgg ctcgattgta cctgcgttca aatactttgc gatcgtgttg cgcgcctgcc 11280
   cggtgcgtcg gctgatctca cggatcgact gcttctctcg caacgccatc cgacggatga 11340
   tgtttaaaag tcccatgtgg atcactccgt tgccccgtcg ctcaccgtgt tggggggaag 11400
   gtgcacatgg ctcagttctc aatggaaatt atctgcctaa ccggctcagt tctgcgtaga 11460
   aaccaacatg caagctccac cgggtgcaaa gcggcagcgg 11500
<210> 4
   <211> 10443
   <212> DNA
   <213> Artificial
<220>
   <223> vector
<400> 4
   agattcgaag ctcggtcccg tgggtgttct gtcgtctcgt tgtacaacga aatccattcc 60
   cattccgcgc tcaagatggc ttcccctcgg cagttcatca gggctaaatc aatctagccg 120
   acttgtccgg tgaaatgggc tgcactccaa cagaaacaat caaacaaaca tacacagcga 180
   cttattcaca cgcgacaaat tacaacggta tatatcctgc cagtactcgg ccgtcgacct 240
   gcaggcaatt ggtacctacg tatgcatggc gcgccatatg caccatacat ggagtcaaaa 300
   attcagatcg aggatctaac agaactcgcc gtgaagactg gcgaacagtt catacagagt 360
   cttttacgac tcaatgacaa gaagaaaatc ttcgtcaaca tggtggagca cgacactctc 420
   gtctactcca agaatatcaa agatacagtc tcagaagacc aaagggctat tgagactttt 480
   caacaaaggg taatatcggg aaacctcctc ggattccatt gcccagctat ctgtcacttc 540
   atcaaaagga cagtagaaaa ggaaggtggc acctacaaat gccatcattg cgataaagga 600
   aaggctatcg ttcaagatgc ctctgccgac agtggtccca aagatggacc cccacccacg 660
   aggagcatcg tggaaaaaga agacgttcca accacgtctt caaagcaagt ggattgatgt 720
   gatatctcca ctgacgtaag ggatgacgca caatcccact atccttcgca agacccttcc 780
   tctatataag gaagttcatt tcatttggag aggactcgag aattaagcaa aagaagaaga 840
   agaagaagtc caaaaccatg gctaaacccc ccaagaagaa gcgcaaggtt aacatcaaaa 900
   aaaaccaggt aatgaacctg ggtccgaact ctaaactgct gaaagaatac aaatcccagc 960
   tgatcgaact gaacatcgaa cagttcgaag caggtatcgg tctgatcctg ggtgatgctt 1020
   acatccgttc tcgtgatgaa ggtaaaacct actgtatgca gttcgagtgg aaaaacaaag 1080
   catacatgga ccacgtatgt ctgctgtacg atcagtgggt actgtccccg ccgcacaaaa 1140
   aagaacgtgt taaccacctg ggtaacctgg taatcacctg gggcgcccag actttcaaac 1200
   accaagcttt caacaaactg gctaacctgt tcatcgttaa caacaaaaaa accatcccga 1260
   acaacctggt tgaaaactac ctgaccccga tgtctctggc atactggttc atggatgatg 1320
   gtggtaaatg ggattacaac aaaaactcta ccaacaaatc gatcgtactg aacacccagt 1380
   ctttcacttt cgaagaagta gaatacctgg ttaagggtct gcgtaacaaa ttccaactga 1440
   actgttacgt aaaaatcaac aaaaacaaac cgatcatcta catcgattct atgtcttacc 1500
   tgatcttcta caacctgatc aaaccgtacc tgatcccgca gatgatgtac aaactgccga 1560
   acactatctc ctccgaaact ttcctgaaat agggctagca agcttggaca cgctgaaatc 1620
   accagtctct ctctacaaat ctatctctct ctattttctc cataataatg tgtgagtagt 1680
   tcccagataa gggaattagg gttcctatag ggtttcgctc atgtgttgag catataagaa 1740
   acccttagta tgtatttgta tttgtaaaat acttctatca ataaaatttc taattcctaa 1800
   aaccaaaatc cagtactaaa atccagatca tgcatggtac agcggccgcg ttaacgcgta 1860
   tactctagag cgatcgccat ggagccattt acaattgaat atatcctgcc gccgctgccg 1920
   ctttgcaccc ggtggagctt gcatgttggt ttctacgcag aactgagccg gttaggcaga 1980
   taatttccat tgagaactga gccatgtgca ccttcccccc aacacggtga gcgacggggc 2040
   aacggagtga tccacatggg acttttaaac atcatccgtc ggatggcgtt gcgagagaag 2100
   cagtcgatcc gtgagatcag ccgacgcacc gggcaggcgc gcaacacgat cgcaaagtat 2160
   ttgaacgcag gtacaatcga gccgacgttc acggtaccgg aacgaccaag caagctagct 2220
   tagtaaagcc ctcgctagat tttaatgcgg atgttgcgat tacttcgcca actattgcga 2280
   taacaagaaa aagccagcct ttcatgatat atctcccaat ttgtgtaggg cttattatgc 2340
   acgcttaaaa ataataaaag cagacttgac ctgatagttt ggctgtgagc aattatgtgc 2400
   ttagtgcatc taacgcttga gttaagccgc gccgcgaagc ggcgtcggct tgaacgaatt 2460
   gttagacatt atttgccgac taccttggtg atctcgcctt tcacgtagtg gacaaattct 2520
   tccaactgat ctgcgcgcga ggccaagcga tcttcttctt gtccaagata agcctgtcta 2580
   gcttcaagta tgacgggctg atactgggcc ggcaggcgct ccattgccca gtcggcagcg 2640
   acatccttcg gcgcgatttt gccggttact gcgctgtacc aaatgcggga caacgtaagc 2700
   actacatttc gctcatcgcc agcccagtcg ggcggcgagt tccatagcgt taaggtttca 2760
   tttagcgcct caaatagatc ctgttcagga accggatcaa agagttcctc cgccgctgga 2820
   cctaccaagg caacgctatg ttctcttgct tttgtcagca agatagccag atcaatgtcg 2880
   atcgtggctg gctcgaagat acctgcaaga atgtcattgc gctgccattc tccaaattgc 2940
   agttcgcgct tagctggata acgccacgga atgatgtcgt cgtgcacaac aatggtgact 3000
   tctacagcgc ggagaatctc gctctctcca ggggaagccg aagtttccaa aaggtcgttg 3060
   atcaaagctc gccgcgttgt ttcatcaagc cttacggtca ccgtaaccag caaatcaata 3120
   tcactgtgtg gcttcaggcc gccatccact gcggagccgt acaaatgtac ggccagcaac 3180
   gtcggttcga gatggcgctc gatgacgcca actacctctg atagttgagt cgatacttcg 3240
   gcgatcaccg cttccctcat gatgtttaac tttgttttag ggcgactgcc ctgctgcgta 3300
   acatcgttgc tgctccataa catcaaacat cgacccacgg cgtaacgcgc ttgctgcttg 3360
   gatgcccgag gcatagactg taccccaaaa aaacagtcat aacaagccat gaaaaccgcc 3420
   actgcgccgt taccaccgct gcgttcggtc aaggttctgg accagttgcg tgagcgcata 3480
   cgctacttgc attacagctt acgaaccgaa caggcttatg tccactgggt tcgtgccttc 3540
   atccgtttcc acggtgtgcg tcacccggca accttgggca gcagcgaagt cgaggcattt 3600
   ctgtcctggc tggcgaacga gcgcaaggtt tcggtctcca cgcatcgtca ggcattggcg 3660
   gccttgctgt tcttctacgg caagtgctgt gcacggatct gccctggctt caggagatcg 3720
   gaagacctcg gccgtccggg cgcttgccgg tggtgctgac cccggatgaa gtggttcgca 3780
   tcctcggttt tctggaaggc gagcatcgtt tgttcgccca gcttctgtat ggaacgggtc 3840
   gacgcgttta atgaccagca cagtcgtgat ggcaaggtca gaatagcgct gaggtctgcc 3900
   tcgtgaagaa ggtgttgctg actcatacca ggcctgaatc gccccatcat ccagccagaa 3960
   agtgagggag ccacggttga tgagagcttt gttgtaggtg gaccagttgg tgattttgaa 4020
   cttttgcttt gccacggaac ggtctgcgtt gtcgggaaga tgcgtgatct gatccttcaa 4080
   ctcagcaaaa gttcgattta ttcaacaaag ccgccgtccc gtcaagtcag cgtaatgctc 4140
   tgccagtgtt acaaccaatt aaccaattct gattagaaaa actcatcgag catcaaatga 4200
   aactgcaatt tattcatatc aggattatca ataccatatt tttgaaaaag ccgtttctgt 4260
   aatgaaggag aaaactcacc gaggcagttc cataggatgg caagatcctg gtatcggtct 4320
   gcgattccga ctcgtccaac atcaatacaa cctattaatt tcccctcgtc aaaaataagg 4380
   ttatcaagtg agaaatcacc atgagtgacg actgaatccg gtgagaatgg caaaagttta 4440
   tgcatttctt tccagacttg ttcaacaggc cagccattac gctcgtcatc aaaatcactc 4500
   gcatcaacca aaccgttatt cattcgtgat tgcgcctgag cgagacgaaa tacgcggctg 4560
   ttaaaaggac aattacaaac aggaatcgaa tgcaaccggc gcaggaacac tgccagcgca 4620
   tcaacaatat tttcacctga atcaggatat tcttctaata cctggaatgc tgtttttccg 4680
   gggatcgcag tggtgagtaa ccatgcatca tcaggagtac ggataaaatg cttgatggtc 4740
   ggaagaggca taaattccgt cagccagttt agtctgacca tctcatctgt aacatcattg 4800
   gcaacgctac ctttgccatg tttcagaaac aactctggcg catcgggctt cccatacaat 4860
   cgatagattg tcgcacctga ttgcccgaca ttatcgcgag cccatttata cccatataaa 4920
   tcagcatcca tgttggaatt taatcgcggc ctcgagcaag acgtttcccg ttgaatatgg 4980
   ctcataacac cccttgtatt actgtttatg taagcagaca gttttattgt tcatgatgat 5040
   atatttttat cttgtgcaat gtaacatcag agattttgag acacaacgtg gctttgttga 5100
   ataaatcgaa cttttgctga gttgaaggat cagatcacgc atcttcccga caacgcagac 5160
   cgttccgtgg caaagcaaaa gttcaaaatc accaactggt ccacctacaa caaagctctc 5220
   atcaaccgtg gctccctcac tttctggctg gatgatgggg cgattcaggc ctggtatgag 5280
   tcagcaacac cttcttcacg aggcagacct cagcgctatt ctgaccttgc catcacgact 5340
   gtgctggtca ttaaacgcgt cgacggatca gtgagggttt gcaactgcgg gtcaaggatc 5400
   tggatttcga tcacggcacg atcatcgtgc gggagggcaa gggctccaag gatcgggcct 5460
   tgatgttacc cgagagcttg gcacccagcc tgcgcgagca gggatcgatc caacccctcc 5520
   gctgctatag tgcagtcggc ttctgacgtt cagtgcagcc gtcttctgaa aacgacatgt 5580
   cgcacaagtc ctaagttacg cgacaggctg ccgccctgcc cttttcctgg cgttttcttg 5640
   tcgcgtgttt tagtcgcata aagtagaata cttgcgacta gaaccggaga cattacgcca 5700
   tgaacaagag cgccgccgct ggcctgctgg gctatgcccg cgtcagcacc gacgaccagg 5760
   acttgaccaa ccaacgggcc gaactgcacg cggccggctg caccaagctg ttttccgaga 5820
   agatcaccgg caccaggcgc gaccgcccgg agctggccag gatgcttgac cacctacgcc 5880
   ctggcgacgt tgtgacagtg accaggctag accgcctggc ccgcagcacc cgcgacctac 5940
   tggacattgc cgagcgcatc caggaggccg gcgcgggcct gcgtagcctg gcagagccgt 6000
   gggccgacac caccacgccg gccggccgca tggtgttgac cgtgttcgcc ggcattgccg 6060
   agttcgagcg ttccctaatc atcgaccgca cccggagcgg gcgcgaggcc gccaaggccc 6120
   gaggcgtgaa gtttggcccc cgccctaccc tcaccccggc acagatcgcg cacgcccgcg 6180
   agctgatcga ccaggaaggc cgcaccgtga aagaggcggc tgcactgctt ggcgtgcatc 6240
   gctcgaccct gtaccgcgca cttgagcgca gcgaggaagt gacgcccacc gaggccaggc 6300
   ggcgcggtgc cttccgtgag gacgcattga ccgaggccga cgccctggcg gccgccgaga 6360
   atgaacgcca agaggaacaa gcatgaaacc gcaccaggac ggccaggacg aaccgttttt 6420
   cattaccgaa gagatcgagg cggagatgat cgcggccggg tacgtgttcg agccgcccgc 6480
   gcacgtctca accgtgcggc tgcatgaaat cctggccggt ttgtctgatg ccaagctggc 6540
   ggcctggccg gccagcttgg ccgctgaaga aaccgagcgc cgccgtctaa aaaggtgatg 6600
   tgtatttgag taaaacagct tgcgtcatgc ggtcgctgcg tatatgatgc gatgagtaaa 6660
   taaacaaata cgcaagggga acgcatgaag gttatcgctg tacttaacca gaaaggcggg 6720
   tcaggcaaga cgaccatcgc aacccatcta gcccgcgccc tgcaactcgc cggggccgat 6780
   gttctgttag tcgattccga tccccagggc agtgcccgcg attgggcggc cgtgcgggaa 6840
   gatcaaccgc taaccgttgt cggcatcgac cgcccgacga ttgaccgcga cgtgaaggcc 6900
   atcggccggc gcgacttcgt agtgatcgac ggagcgcccc aggcggcgga cttggctgtg 6960
   tccgcgatca aggcagccga cttcgtgctg attccggtgc agccaagccc ttacgacata 7020
   tgggccaccg ccgacctggt ggagctggtt aagcagcgca ttgaggtcac ggatggaagg 7080
   ctacaagcgg cctttgtcgt gtcgcgggcg atcaaaggca cgcgcatcgg cggtgaggtt 7140
   gccgaggcgc tggccgggta cgagctgccc attcttgagt cccgtatcac gcagcgcgtg 7200
   agctacccag gcactgccgc cgccggcaca accgttcttg aatcagaacc cgagggcgac 7260
   gctgcccgcg aggtccaggc gctggccgct gaaattaaat caaaactcat ttgagttaat 7320
   gaggtaaaga gaaaatgagc aaaagcacaa acacgctaag tgccggccgt ccgagcgcac 7380
   gcagcagcaa ggctgcaacg ttggccagcc tggcagacac gccagccatg aagcgggtca 7440
   actttcagtt gccggcggag gatcacacca agctgaagat gtacgcggta cgccaaggca 7500
   agaccattac cgagctgcta tctgaataca tcgcgcagct accagagtaa atgagcaaat 7560
   gaataaatga gtagatgaat tttagcggct aaaggaggcg gcatggaaaa tcaagaacaa 7620
   ccaggcaccg acgccgtgga atgccccatg tgtggaggaa cgggcggttg gccaggcgta 7680
   agcggctggg ttgtctgccg gccctgcaat ggcactggaa cccccaagcc cgaggaatcg 7740
   gcgtgacggt cgcaaaccat ccggcccggt acaaatcggc gcggcgctgg gtgatgacct 7800
   ggtggagaag ttgaaggccg cgcaggccgc ccagcggcaa cgcatcgagg cagaagcacg 7860
   ccccggtgaa tcgtggcaag cggccgctga tcgaatccgc aaagaatccc ggcaaccgcc 7920
   ggcagccggt gcgccgtcga ttaggaagcc gcccaagggc gacgagcaac cagatttttt 7980
   cgttccgatg ctctatgacg tgggcacccg cgatagtcgc agcatcatgg acgtggccgt 8040
   tttccgtctg tcgaagcgtg accgacgagc tggcgaggtg atccgctacg agcttccaga 8100
   cgggcacgta gaggtttccg cagggccggc cggcatggcc agtgtgtggg attacgacct 8160
   ggtactgatg gcggtttccc atctaaccga atccatgaac cgataccggg aagggaaggg 8220
   agacaagccc ggccgcgtgt tccgtccaca cgttgcggac gtactcaagt tctgccggcg 8280
   agccgatggc ggaaagcaga aagacgacct ggtagaaacc tgcattcggt taaacaccac 8340
   gcacgttgcc atgcagcgta cgaagaaggc caagaacggc cgcctggtga cggtatccga 8400
   gggtgaagcc ttgattagcc gctacaagat cgtaaagagc gaaaccgggc ggccggagta 8460
   catcgagatc gagctagctg attggatgta ccgcgagatc acagaaggca agaacccgga 8520
   cgtgctgacg gttcaccccg attacttttt gatcgatccc ggcatcggcc gttttctcta 8580
   ccgcctggca cgccgcgccg caggcaaggc agaagccaga tggttgttca agacgatcta 8640
   cgaacgcagt ggcagcgccg gagagttcaa gaagttctgt ttcaccgtgc gcaagctgat 8700
   cgggtcaaat gacctgccgg agtacgattt gaaggaggag gcggggcagg ctggcccgat 8760
   cctagtcatg cgctaccgca acctgatcga gggcgaagca tccgccggtt cctaatgtac 8820
   ggagcagatg ctagggcaaa ttgccctagc aggggaaaaa ggtcgaaaag gtctctttcc 8880
   tgtggatagc acgtacattg ggaacccaaa gccgtacatt gggaaccgga acccgtacat 8940
   tgggaaccca aagccgtaca ttgggaaccg gtcacacatg taagtgactg atataaaaga 9000
   gaaaaaaggc gatttttccg cctaaaactc tttaaaactt attaaaactc ttaaaacccg 9060
   cctggcctgt gcataactgt ctggccagcg cacagccgaa gagctgcaaa aagcgcctac 9120
   ccttcggtcg ctgcgctccc tacgccccgc cgcttcgcgt cggcctatcg cggccgctgg 9180
   ccgctcaaaa atggctggcc tacggccagg caatctacca gggcgcggac aagccgcgcc 9240
   gtcgccactc gaccgccggc gcccacatca aggcaccctg cctcgcgcgt ttcggtgatg 9300
   acggtgaaaa cctctgacac atgcagctcc cggagacggt cacagcttgt ctgtaagcgg   9360
   atgccgggag cagacaagcc cgtcagggcg cgtcagcggg tgttggcggg tgtcggggcg   9420
   cagccatgac ccagtcacgt agcgatagcg gagtgtatac tggcttaact atgcggcatc   9480
   agagcagatt gtactgagag tgcaccatat gcggtgtgaa ataccgcaca gatgcgtaag   9540
   gagaaaatac cgcatcaggc gctcttccgc ttcctcgctc actgactcgc tgcgctcggt   9600
   cgttcggctg cggcgagcgg tatcagctca ctcaaaggcg gtaatacggt tatccacaga   9660
   atcaggggat aacgcaggaa agaacatgtg agcaaaaggc cagcaaaagg ccaggaaccg   9720
   taaaaaggcc gcgttgctgg cgtttttcca taggctccgc ccccctgacg agcatcacaa   9780
   aaatcgacgc tcaagtcaga ggtggcgaaa cccgacagga ctataaagat accaggcgtt   9840
   tccccctgga agctccctcg tgcgctctcc tgttccgacc ctgccgctta ccggatacct   9900
   gtccgccttt ctcccttcgg gaagcgtggc gctttctcat agctcacgct gtaggtatct   9960
   cagttcggtg taggtcgttc gctccaagct gggctgtgtg cacgaacccc ccgttcagcc  10020
   cgaccgctgc gccttatccg gtaactatcg tcttgagtcc aacccggtaa gacacgactt  10080
   atcgccactg gcagcagcca ctggtaacag gattagcaga gcgaggtatg taggcggtgc  10140
   tacagagttc ttgaagtggt ggcctaacta cggctacact agaaggacag tatttggtat  10200
   ctgcgctctg ctgaagccag ttaccttcgg aaaaagagtt ggtagctctt gatccggcaa  10260
   acaaaccacc gctggtagcg gtggtttttt tgtttgcaag cagcagatta cgcgcagaaa  10320
   aaaaggatct caagaagatc cggaaaacgc aagcgcaaag agaaagcagg tagcttgcag  10380
   tgggcttaca tggcgatagc tagactgggc ggttttatgg acagcaagcg aaccggaatt  10440
   gcc                                                                10443